# EUROPÄISCHE PATENTSCHRIFT

(11) **EP 2 270 033 B1**
(45) Veröffentlichungstag und Bekanntmachung des Hinweises auf die Patenterteilung: **17.02.2016**
(21) Anmeldenummer: 10168382.9
(22) Anmeldetag: 03.07.2010
(51) Int. Cl.: C07K 14/32

(54) **Mikroröhren, umfassend Bestandteile der äußeren Membran von E. coli Zellen und rekombinant exprimierte S-Layer-Proteine, Verfahren zur Herstellung und Verwendung**
Microtubes comprising components of the external membrane of E. coli cells and recombinant S-layer proteins, method for producing same and application
Micro-tubes comprenant des composants de la membrane extérieure de cellules d'E. coli et de protéines à couche S recombinantes exprimées, leur procédé de fabrication et d'utilisation

(30) Priorität: 03.07.2009 DE 102009032645
(43) Veröffentlichungstag der Anmeldung: 05.01.2011
(73) Patentinhaber: Helmholtz-Zentrum Dresden - Rossendorf e.V., 01328 Dresden (DE)
(72) Erfinder: Pollmann, Katrin, Dr., 01324 Dresden (DE); Lederer, Franziska, 01920 Oßling OT Weißig (DE); Raff, Johannes, Dr., 01324 Dresden (DE)
(74) Vertreter: Kailuweit & Uhlemann Patentanwälte Partnerschaft mbB

(56) Entgegenhaltungen:
- EP-A1- 2 101 175
- POLLMANN KATRIN ET AL: "Construction of an S-layer protein exhibiting modified self-assembling properties and enhanced metal binding capacities." APPLIED MICROBIOLOGY AND BIOTECHNOLOGY JUL 2007 LNKD- PUBMED:17437097, Bd. 75, Nr. 5, Juli 2007 (2007-07), Seiten 1079-1085, XP019513753 ISSN: 0175-7598
- POLLMANN KATRIN ET AL: "Novel surface layer protein genes in Bacillus sphaericus associated with unusual insertion elements." MICROBIOLOGY (READING, ENGLAND) SEP 2005 LNKD- PUBMED:16151207, Bd. 151, Nr. Pt 9, September 2005 (2005-09), Seiten 2961-2973, XP009138823 ISSN: 1350-0872
- SLEYTR UWE B ET AL: "S-layers as a tool kit for nanobiotechnological applications" FEMS MICROBIOLOGY LETTERS, BLACKWELL PUBLISHING, AMSTERDAM, NL LNKD- DOI:J.1574-6968.2006.00573.X, Bd. 267, Nr. 2, 1. Februar 2007 (2007-02-01), Seiten 131-144, XP002529465 ISSN: 0378-1097 [gefunden am 2007-01-04]

### SEQUENCE LISTING

<110> Forschungszentrum Dresden-Rossendorf
<120> Mikroröhren, umfassend Bestandteile der äußeren Membran von E. coli
   Zellen und rekombinant exprimierte S-Layer-Proteine, Verfahren zu ihrer Herstellung und Verwendung
<130> P0903EP
<150> DE 10 2009 032 645.6
   <151> 2009-07-03
<160> 15
<170> PatentIn version 3.5
<210> 1
   <211> 3684
   <212> DNA
   <213> Lysinibacillus sphaericus
<220>
   <221> CDS
   <222> (1)..(3684)
<220>
   <221> misc_feature
   <222> (411)..(411)
   <223> n is a, c, g, or t
<400> 1
   atg gct aac caa cca aag aaa tac aaa aaa ttc gta gcg acg gca gca       48
   Met Ala Asn Gln Pro Lys Lys Tyr Lys Lys Phe Val Ala Thr Ala Ala
   1               5                   10                  15
   acg gca aca tta gta gca tct gct atc gta cca gtg gtt tct gcc gca       96
   Thr Ala Thr Leu Val Ala Ser Ala Ile Val Pro Val Val Ser Ala Ala
               20                  25                  30
   gga ttt tca gat gta gca ggt aac gac cac gaa gta gca atc aac gca      144
   Gly Phe Ser Asp Val Ala Gly Asn Asp His Glu Val Ala Ile Asn Ala
           35                  40                  45
   ctt gct gat gca ggt atc atc aat gga tac gct gac ggc aca ttc aaa      192
   Leu Ala Asp Ala Gly Ile Ile Asn Gly Tyr Ala Asp Gly Thr Phe Lys
       50                  55                  60
   cca aac caa aca att aac cgc ggt caa gtg gtt aaa tta tta ggt cgt      240
   Pro Asn Gln Thr Ile Asn Arg Gly Gln Val Val Lys Leu Leu Gly Arg
   65                  70                  75                  80
   tat tta gaa gca caa ggc caa gaa att cca gct gac tgg aat tca aaa      288
   Tyr Leu Glu Ala Gln Gly Gln Glu Ile Pro Ala Asp Trp Asn Ser Lys
                   85                  90                  95
   caa cgc ttc aac gat cta cca gta aca gct gaa gca gaa tta gta aaa      336
   Gln Arg Phe Asn Asp Leu Pro Val Thr Ala Glu Ala Glu Leu Val Lys
               100                 105                 110
   tat gct gca cta gca aaa gat gca ggc gta ttc aac ggt tca aac ggc      384
   Tyr Ala Ala Leu Ala Lys Asp Ala Gly Val Phe Asn Gly Ser Asn Gly
           115                 120                 125
   aac tta aac gct tca caa aca atg can cgt caa caa atg gca gta gtt      432
   Asn Leu Asn Ala Ser Gln Thr Met Xaa Arg Gln Gln Met Ala Val Val
       130                 135                 140
   tta gta cgt gct att aaa gaa atc gct ggc gta gac tta gta gct gag       480
   Leu Val Arg Ala Ile Lys Glu Ile Ala Gly Val Asp Leu Val Ala Glu
   145                 150                 155                 160
   tac aaa aaa gcg aat ttc gta aca gaa att ggt gac tta gac aaa gct       528
   Tyr Lys Lys Ala Asn Phe Val Thr Glu Ile Gly Asp Leu Asp Lys Ala
                   165                 170                 175
   tac tct gca gaa caa cgc aca gcg atc gtt gct tta gaa tat gcg ggt       576
   Tyr Ser Ala Glu Gln Arg Thr Ala Ile Val Ala Leu Glu Tyr Ala Gly
               180                 185                 190
   att aca aat gtt gct cac ttc aac cca ggt aac agc gta aca cgc gga       624
   Ile Thr Asn Val Ala His Phe Asn Pro Gly Asn Ser Val Thr Arg Gly
           195                 200                 205
   caa ttc gct tca ttc tta tac cgc aca atc gaa aac gtg gta aac gct       672
   Gln Phe Ala Ser Phe Leu Tyr Arg Thr Ile Glu Asn Val Val Asn Ala
       210                 215                 220
   cct gaa gct ggc gct gcg gca gtg aaa gct gta aac aac aca act gtt       720
   Pro Glu Ala Gly Ala Ala Ala Val Lys Ala Val Asn Asn Thr Thr Val
   225                 230                 235                 240
   gaa gta aca ttc gac gaa gaa gta gac aac gta caa gct ctt aac ttc       768
   Glu Val Thr Phe Asp Glu Glu Val Asp Asn Val Gln Ala Leu Asn Phe
                   245                 250                 255
   tta atc tct gat tta gaa gtg aaa aac gca gct gta aaa caa act aac       816
   Leu Ile Ser Asp Leu Glu Val Lys Asn Ala Ala Val Lys Gln Thr Asn
               260                 265                 270
   aaa aaa gtt gtt gtt tta act act gca cct caa act gct gac aaa gag       864
   Lys Lys Val Val Val Leu Thr Thr Ala Pro Gln Thr Ala Asp Lys Glu
           275                 280                 285
   tac act gta tct ctt ggc gaa gaa aaa att ggt act ttc aaa ggt atc       912
   Tyr Thr Val Ser Leu Gly Glu Glu Lys Ile Gly Thr Phe Lys Gly Ile
       290                 295                 300
   gca gct gta gtt cct act aaa gta gat tta gta gaa aaa tca gtt caa       960
   Ala Ala Val Val Pro Thr Lys Val Asp Leu Val Glu Lys Ser Val Gln
   305                 310                 315                 320
   ggt aaa ctt ggc caa caa gta aca ctg aaa gca caa gta aca gtt gct      1008
   Gly Lys Leu Gly Gln Gln Val Thr Leu Lys Ala Gln Val Thr Val Ala
                   325                 330                 335
   gaa ggt caa aca aaa gct ggt att cca gta act ttc ttc atc cca ggt      1056
   Glu Gly Gln Thr Lys Ala Gly Ile Pro Val Thr Phe Phe Ile Pro Gly
               340                 345                 350
   tct gct aat ggt gtg aaa tct cca gta aca gtt gaa gct gta aca gat      1104
   Ser Ala Asn Gly Val Lys Ser Pro Val Thr Val Glu Ala Val Thr Asp
           355                 360                 365
   gaa aac ggt gtt gct tct tat act tac act cgt tac gct gca aca aac      1152
   Glu Asn Gly Val Ala Ser Tyr Thr Tyr Thr Arg Tyr Ala Ala Thr Asn
       370                 375                 380
   gac aca gta act gta tac gcg aac ggt gac cgt tct aaa ttc tca act      1200
   Asp Thr Val Thr Val Tyr Ala Asn Gly Asp Arg Ser Lys Phe Ser Thr
   385                 390                 395                 400
   ggt tat gtc ttc tgg gct gtt gat aca act cta gaa atc act gaa gta      1248
   Gly Tyr Val Phe Trp Ala Val Asp Thr Thr Leu Glu Ile Thr Glu Val
                   405                 410                 415
   act act ggc gat att atc aac aac ggt gct aac aaa act tat aaa gtt      1296
   Thr Thr Gly Asp Ile Ile Asn Asn Gly Ala Asn Lys Thr Tyr Lys Val
               420                 425                 430
   gca tat aaa gac cca gaa act ggt aaa cca gta tca ggt aaa gta ctt      1344
   Ala Tyr Lys Asp Pro Glu Thr Gly Lys Pro Val Ser Gly Lys Val Leu
           435                 440                 445
   aac gtt tct gta aaa gaa aac att gac gta act gtt gat aaa tta caa      1392
   Asn Val Ser Val Lys Glu Asn Ile Asp Val Thr Val Asp Lys Leu Gln
       450                 455                 460
   aac gtt aca att aat ggt att aaa gta gct caa act agc act tca aat      1440
   Asn Val Thr Ile Asn Gly Ile Lys Val Ala Gln Thr Ser Thr Ser Asn
   465                 470                 475                 480
   act aca gct gca caa gtt aca act gac tct aaa ggt gaa gca aca ttc      1488
   Thr Thr Ala Ala Gln Val Thr Thr Asp Ser Lys Gly Glu Ala Thr Phe
                   485                 490                 495
   act att act ggc gca aat gct gaa gta act cca gtt gta ttt gct ggc      1536
   Thr Ile Thr Gly Ala Asn Ala Glu Val Thr Pro Val Val Phe Ala Gly
               500                 505                 510
   gaa gca gtt act ggt caa aca tta cct tct cag aaa tat aat gct gat      1584
   Glu Ala Val Thr Gly Gln Thr Leu Pro Ser Gln Lys Tyr Asn Ala Asp
           515                 520                 525
   gca tta caa aca act gct aaa aag gta aaa ttc ggt gcg ctt caa gct      1632
   Ala Leu Gln Thr Thr Ala Lys Lys Val Lys Phe Gly Ala Leu Gln Ala
       530                 535                 540
   gac tat att ctt gaa gta act cgt gac ggt ggc gaa aca gct gct act      1680
   Asp Tyr Ile Leu Glu Val Thr Arg Asp Gly Gly Glu Thr Ala Ala Thr
   545                 550                 555                 560
   gga tat gat aac gga cgt aaa tat aaa ctt gtc gtt aaa gat aaa aac      1728
   Gly Tyr Asp Asn Gly Arg Lys Tyr Lys Leu Val Val Lys Asp Lys Asn
                   565                 570                 575
   ggt aaa tta gca gca aat gaa aca gta aat gtt gca ttt aac gaa gat      1776
   Gly Lys Leu Ala Ala Asn Glu Thr Val Asn Val Ala Phe Asn Glu Asp
               580                 585                 590
   atc gat ggc gtt atc tct aca act act gat gct aaa ttt gtt aaa tta      1824
   Ile Asp Gly Val Ile Ser Thr Thr Thr Asp Ala Lys Phe Val Lys Leu
           595                 600                 605
   gat aac gaa gac aat caa gtt ggt tgg gat act act gta gat aaa gat      1872
   Asp Asn Glu Asp Asn Gln Val Gly Trp Asp Thr Thr Val Asp Lys Asp
       610                 615                 620
   gct aag aaa atc act gtt aaa aca gat tca aaa ggt gaa gct agc ttc      1920
   Ala Lys Lys Ile Thr Val Lys Thr Asp Ser Lys Gly Glu Ala Ser Phe
   625                 630                 635                 640
   att atc ggt aat act aaa gaa aac aca tat gst aca cca atc gct tgg      1968
   Ile Ile Gly Asn Thr Lys Glu Asn Thr Tyr Xaa Thr Pro Ile Ala Trp
                   645                 650                 655
   att gat gtt aac tat caa agt ggt aaa gtt gga aca tta gac caa gga      2016
   Ile Asp Val Asn Tyr Gln Ser Gly Lys Val Gly Thr Leu Asp Gln Gly
               660                 665                 670
   gag cca agt gca act gct cca atc tct tac ttc caa tct cca act att      2064
   Glu Pro Ser Ala Thr Ala Pro Ile Ser Tyr Phe Gln Ser Pro Thr Ile
           675                 680                 685
   gat ggt tct aaa tta gtt gca gaa aat aaa gat ggt aaa aaa cct tct      2112
   Asp Gly Ser Lys Leu Val Ala Glu Asn Lys Asp Gly Lys Lys Pro Ser
       690                 695                 700
   gat ttt gaa gat aaa gat gct atc ttt aaa gta gta ctt gta aac caa      2160
   Asp Phe Glu Asp Lys Asp Ala Ile Phe Lys Val Val Leu Val Asn Gln
   705                 710                 715                 720
   agt ggt aaa gaa atg aaa aac cac aat tac aaa aca gaa aaa gtt tct      2208
   Ser Gly Lys Glu Met Lys Asn His Asn Tyr Lys Thr Glu Lys Val Ser
                   725                 730                 735
   tat acg gtg aat aac act ggt tct aat gag gct gtt gta gag tac aca      2256
   Tyr Thr Val Asn Asn Thr Gly Ser Asn Glu Ala Val Val Glu Tyr Thr
               740                 745                 750
   tat aac ggt aaa gtt gag tca gaa aca ctt gct cca aat cgt gct act      2304
   Tyr Asn Gly Lys Val Glu Ser Glu Thr Leu Ala Pro Asn Arg Ala Thr
           755                 760                 765
   tct gta gtt gct gat aat ggt aca att aca gtt aaa cca aat ggt aaa      2352
   Ser Val Val Ala Asp Asn Gly Thr Ile Thr Val Lys Pro Asn Gly Lys
       770                 775                 780
   act act tct gta aaa gtt cta gca act ggt gtt gca aaa gaa gat aaa      2400
   Thr Thr Ser Val Lys Val Leu Ala Thr Gly Val Ala Lys Glu Asp Lys
   785                 790                 795                 800
   act aat ggt aaa gaa tat gca ttc act gct aaa gaa gca act gca aaa      2448
   Thr Asn Gly Lys Glu Tyr Ala Phe Thr Ala Lys Glu Ala Thr Ala Lys
                   805                 810                 815
   ttc aca gct act aaa gaa att tca aac cca tat act ggt gcg att aaa      2496
   Phe Thr Ala Thr Lys Glu Ile Ser Asn Pro Tyr Thr Gly Ala Ile Lys
               820                 825                 830
   tac tac gat act gat aag aaa act atc act ttc gat ggt aaa gac gca      2544
   Tyr Tyr Asp Thr Asp Lys Lys Thr Ile Thr Phe Asp Gly Lys Asp Ala
           835                 840                 845
   atc aaa tat gct ggt gaa tct ggt aag aaa tat aaa tac tac agc ggt      2592
   Ile Lys Tyr Ala Gly Glu Ser Gly Lys Lys Tyr Lys Tyr Tyr Ser Gly
       850                 855                 860
   aac att gaa gtt gca aca gaa aaa gca ttt att gat tta tta gct aat      2640
   Asn Ile Glu Val Ala Thr Glu Lys Ala Phe Ile Asp Leu Leu Ala Asn
   865                 870                 875                 880
   gct tct gga aaa gta act gtt act tat aaa gtt gaa gat gat act aaa      2688
   Ala Ser Gly Lys Val Thr Val Thr Tyr Lys Val Glu Asp Asp Thr Lys
                   885                 890                 895
   tca ttc cat att att aac gtt gga act gat gta gct aat aaa cct gta      2736
   Ser Phe His Ile Ile Asn Val Gly Thr Asp Val Ala Asn Lys Pro Val
               900                 905                 910
   gaa aag aaa gat gat aca aaa cct aca aac aat gca cct gtt gct aaa      2784
   Glu Lys Lys Asp Asp Thr Lys Pro Thr Asn Asn Ala Pro Val Ala Lys
           915                 920                 925
   caa gtt gct gat caa gtt att gcg tct aat gga agt aaa tta aca ttt      2832
   Gln Val Ala Asp Gln Val Ile Ala Ser Asn Gly Ser Lys Leu Thr Phe
       930                 935                 940
   aca gct aat gat gta gca tca gat gct gat aaa gat act tta aaa att      2880
   Thr Ala Asn Asp Val Ala Ser Asp Ala Asp Lys Asp Thr Leu Lys Ile
   945                 950                 955                 960
   gtt act gca tat act aca aat tct aca gta gca act gca act act gat      2928
   Val Thr Ala Tyr Thr Thr Asn Ser Thr Val Ala Thr Ala Thr Thr Asp
                       965                 970                 975
   gct aac caa tta ggt ttc tct gta gaa cca aaa ggt gaa ggt tca aca      2976
   Ala Asn Gln Leu Gly Phe Ser Val Glu Pro Lys Gly Glu Gly Ser Thr
                   980                 985                 990
   act gta act gtt gta gtt act gat  ggt aaa gat aat att  aat gta agc    3024
   Thr Val Thr Val Val Val Thr Asp  Gly Lys Asp Asn Ile  Asn Val Ser
           995                 1000                 1005
   ttc aag  gtt aat gta tct act  gct agc tat act tct  gta gta aac       3069
   Phe Lys  Val Asn Val Ser Thr  Ala Ser Tyr Thr Ser  Val Val Asn
       1010                 1015                 1020
   tct ggt  aac cca gtt aaa gat  ttt gct cct gca gtt  cct aca gca       3114
   Ser Gly  Asn Pro Val Lys Asp  Phe Ala Pro Ala Val  Pro Thr Ala
       1025                 1030                 1035
   gca aca  gca aaa cta cca gaa  ttc act tct gta gca  aaa gct ggt       3159
   Ala Thr  Ala Lys Leu Pro Glu  Phe Thr Ser Val Ala  Lys Ala Gly
       1040                 1045                 1050
   act att  aaa gta caa gga ttt  gat att cct tta aca  ctt ggt tct       3204
   Thr Ile  Lys Val Gln Gly Phe  Asp Ile Pro Leu Thr  Leu Gly Ser
       1055                 1060                 1065
   act caa  gca caa gtg cta gag  aca ctt aat aat ttt  att act gac       3249
   Thr Gln  Ala Gln Val Leu Glu  Thr Leu Asn Asn Phe  Ile Thr Asp
       1070                 1075                 1080
   tat aaa  att aat gca agt gct  aaa ctt gtt gat aaa  gcg att gta       3294
   Tyr Lys  Ile Asn Ala Ser Ala  Lys Leu Val Asp Lys  Ala Ile Val
       1085                 1090                 1095
   att agc  tct act gaa aca ggt  aac gct gct act cta  aca gtt gaa       3339
   Ile Ser  Ser Thr Glu Thr Gly  Asn Ala Ala Thr Leu  Thr Val Glu
       1100                 1105                 1110
   ggt gat  agt aca tta gaa ctt  gta gct ggt act gta  tta ggt act       3384
   Gly Asp  Ser Thr Leu Glu Leu  Val Ala Gly Thr Val  Leu Gly Thr
       1115                 1120                 1125
   gtt tca  act aca cct ggt tca  gtt ggt act gct act  cct gct aaa       3429
   Val Ser  Thr Thr Pro Gly Ser  Val Gly Thr Ala Thr  Pro Ala Lys
       1130                 1135                 1140
   tac gct  gta aaa gta tta aac  ggt gta agt aac gga  aca aaa gtt       3474
   Tyr Ala  Val Lys Val Leu Asn  Gly Val Ser Asn Gly  Thr Lys Val
       1145                 1150                 1155
   gat ttc  aaa ttt aca att ggt  gca aaa aca gta aca  gta act gtt       3519
   Asp Phe  Lys Phe Thr Ile Gly  Ala Lys Thr Val Thr  Val Thr Val
       1160                 1165                 1170
   gat gca  act gct ggt gca aaa  gca gta tct gac gtt  gta gat act       3564
   Asp Ala  Thr Ala Gly Ala Lys  Ala Val Ser Asp Val  Val Asp Thr
       1175                 1180                 1185
   tta gct  gct gca aca tta gat  gga tac tca atc act  aaa aat gga       3609
   Leu Ala  Ala Ala Thr Leu Asp  Gly Tyr Ser Ile Thr  Lys Asn Gly
       1190                 1195                 1200
   gat gtt  att gag tta act caa  aca act cca gca aca  act act gat       3654
   Asp Val  Ile Glu Leu Thr Gln  Thr Thr Pro Ala Thr  Thr Thr Asp
       1205                 1210                 1215
   aaa tta  gtt gtt gaa act aaa  aaa gct aac                           3684
   Lys Leu  Val Val Glu Thr Lys  Lys Ala Asn
       1220                 1225
<210> 2
   <211> 1228
   <212> PRT
   <213> Lysinibacillus sphaericus
<220>
   <221> misc_feature
   <222> (137)..(137)
   <223> The 'xaa' at location 137 stands for Gln, or His.
<220>
   <221> misc_feature
   <222> (651)..(651)
   <223> The 'Xaa' at location 651 stands for Gly, or Ala.
<400> 2
   Met Ala Asn Gln Pro Lys Lys Tyr Lys Lys Phe Val Ala Thr Ala Ala
   1               5                   10                  15
   Thr Ala Thr Leu Val Ala Ser Ala Ile Val Pro Val Val Ser Ala Ala
               20                  25                  30
   Gly Phe Ser Asp Val Ala Gly Asn Asp His Glu Val Ala Ile Asn Ala
           35                  40                  45
   Leu Ala Asp Ala Gly Ile Ile Asn Gly Tyr Ala Asp Gly Thr Phe Lys
       50                  55                  60
   Pro Asn Gln Thr Ile Asn Arg Gly Gln Val Val Lys Leu Leu Gly Arg
   65                  70                  75                  80
   Tyr Leu Glu Ala Gln Gly Gln Glu Ile Pro Ala Asp Trp Asn Ser Lys
                   85                  90                  95
   Gln Arg Phe Asn Asp Leu Pro Val Thr Ala Glu Ala Glu Leu Val Lys
               100                 105                 110
   Tyr Ala Ala Leu Ala Lys Asp Ala Gly Val Phe Asn Gly Ser Asn Gly
           115                 120                 125
   Asn Leu Asn Ala Ser Gln Thr Met Xaa Arg Gln Gln Met Ala Val Val
       130                 135                 140
   Leu Val Arg Ala Ile Lys Glu Ile Ala Gly Val Asp Leu Val Ala Glu
   145                 150                 155                 160
   Tyr Lys Lys Ala Asn Phe Val Thr Glu Ile Gly Asp Leu Asp Lys Ala
                   165                 170                 175
   Tyr Ser Ala Glu Gln Arg Thr Ala Ile Val Ala Leu Glu Tyr Ala Gly
               180                 185                 190
   Ile Thr Asn Val Ala His Phe Asn Pro Gly Asn Ser Val Thr Arg Gly
           195                 200                 205
   Gln Phe Ala Ser Phe Leu Tyr Arg Thr Ile Glu Asn Val Val Asn Ala
       210                 215                 220
   Pro Glu Ala Gly Ala Ala Ala Val Lys Ala Val Asn Asn Thr Thr Val
   225                 230                 235                     240
   Glu Val Thr Phe Asp Glu Glu Val Asp Asn Val Gln Ala Leu Asn Phe
                   245                 250                 255
   Leu Ile Ser Asp Leu Glu Val Lys Asn Ala Ala Val Lys Gln Thr Asn
               260                 265                 270
   Lys Lys Val Val Val Leu Thr Thr Ala Pro Gln Thr Ala Asp Lys Glu
           275                 280                 285
   Tyr Thr Val Ser Leu Gly Glu Glu Lys Ile Gly Thr Phe Lys Gly Ile
       290                 295                 300
   Ala Ala Val Val Pro Thr Lys Val Asp Leu Val Glu Lys Ser Val Gln
   305                 310                 315                     320
   Gly Lys Leu Gly Gln Gln Val Thr Leu Lys Ala Gln Val Thr Val Ala
                   325                 330                 335
   Glu Gly Gln Thr Lys Ala Gly Ile Pro Val Thr Phe Phe Ile Pro Gly
               340                 345                 350
   Ser Ala Asn Gly Val Lys Ser Pro Val Thr Val Glu Ala Val Thr Asp
           355                 360                 365
   Glu Asn Gly Val Ala Ser Tyr Thr Tyr Thr Arg Tyr Ala Ala Thr Asn
       370                 375                 380
   Asp Thr Val Thr Val Tyr Ala Asn Gly Asp Arg Ser Lys Phe Ser Thr
   385                 390                 395                     400
   Gly Tyr Val Phe Trp Ala Val Asp Thr Thr Leu Glu Ile Thr Glu Val
                   405                 410                 415
   Thr Thr Gly Asp Ile Ile Asn Asn Gly Ala Asn Lys Thr Tyr Lys Val
               420                 425                 430
   Ala Tyr Lys Asp Pro Glu Thr Gly Lys Pro Val Ser Gly Lys Val Leu
           435                 440                 445
   Asn Val Ser Val Lys Glu Asn Ile Asp Val Thr Val Asp Lys Leu Gln
       450                 455                 460
   Asn Val Thr Ile Asn Gly Ile Lys Val Ala Gln Thr Ser Thr Ser Asn
   465                 470                 475                     480
   Thr Thr Ala Ala Gln Val Thr Thr Asp Ser Lys Gly Glu Ala Thr Phe
   485 490 495
   Thr Ile Thr Gly Ala Asn Ala Glu Val Thr Pro Val Val Phe Ala Gly
   500 505 510
   Glu Ala Val Thr Gly Gln Thr Leu Pro Ser Gln Lys Tyr Asn Ala Asp
   515 520 525
   Ala Leu Gln Thr Thr Ala Lys Lys Val Lys Phe Gly Ala Leu Gln Ala
   530 535 540
   Asp Tyr Ile Leu Glu Val Thr Arg Asp Gly Gly Glu Thr Ala Ala Thr
   545 550 555 560
   Gly Tyr Asp Asn Gly Arg Lys Tyr Lys Leu Val Val Lys Asp Lys Asn
   565 570 575
   Gly Lys Leu Ala Ala Asn Glu Thr Val Asn Val Ala Phe Asn Glu Asp
   580 585 590
   Ile Asp Gly Val Ile Ser Thr Thr Thr Asp Ala Lys Phe Val Lys Leu
   595 600 605
   Asp Asn Glu Asp Asn Gln Val Gly Trp Asp Thr Thr Val Asp Lys Asp
   610 615 620
   Ala Lys Lys Ile Thr Val Lys Thr Asp Ser Lys Gly Glu Ala Ser Phe
   625 630 635 640
   Ile Ile Gly Asn Thr Lys Glu Asn Thr Tyr Xaa Thr Pro Ile Ala Trp
   645 650 655
   Ile Asp Val Asn Tyr Gln Ser Gly Lys Val Gly Thr Leu Asp Gln Gly
   660 665 670
   Glu Pro Ser Ala Thr Ala Pro Ile Ser Tyr Phe Gln Ser Pro Thr Ile
   675 680 685
   Asp Gly Ser Lys Leu Val Ala Glu Asn Lys Asp Gly Lys Lys Pro Ser
   690 695 700
   Asp Phe Glu Asp Lys Asp Ala Ile Phe Lys Val Val Leu Val Asn Gln
   705 710 715 720
   Ser Gly Lys Glu Met Lys Asn His Asn Tyr Lys Thr Glu Lys Val Ser
   725 730 735
   Tyr Thr Val Asn Asn Thr Gly Ser Asn Glu Ala Val Val Glu Tyr Thr
   740 745 750
   Tyr Asn Gly Lys Val Glu Ser Glu Thr Leu Ala Pro Asn Arg Ala Thr
   755 760 765
   Ser Val Val Ala Asp Asn Gly Thr Ile Thr Val Lys Pro Asn Gly Lys
   770 775 780
   Thr Thr Ser Val Lys Val Leu Ala Thr Gly Val Ala Lys Glu Asp Lys
   785 790 795 800
   Thr Asn Gly Lys Glu Tyr Ala Phe Thr Ala Lys Glu Ala Thr Ala Lys
   805 810 815
   Phe Thr Ala Thr Lys Glu Ile Ser Asn Pro Tyr Thr Gly Ala Ile Lys
   820 825 830
   Tyr Tyr Asp Thr Asp Lys Lys Thr Ile Thr Phe Asp Gly Lys Asp Ala
   835 840 845
   Ile Lys Tyr Ala Gly Glu Ser Gly Lys Lys Tyr Lys Tyr Tyr Ser Gly
   850 855 860
   Asn Ile Glu Val Ala Thr Glu Lys Ala Phe Ile Asp Leu Leu Ala Asn
   865 870 875 880
   Ala Ser Gly Lys Val Thr Val Thr Tyr Lys Val Glu Asp Asp Thr Lys
   885 890 895
   Ser Phe His Ile Ile Asn Val Gly Thr Asp Val Ala Asn Lys Pro Val
   900 905 910
   Glu Lys Lys Asp Asp Thr Lys Pro Thr Asn Asn Ala Pro Val Ala Lys
   915 920 925
   Gln Val Ala Asp Gln Val Ile Ala Ser Asn Gly Ser Lys Leu Thr Phe
   930 935 940
   Thr Ala Asn Asp Val Ala Ser Asp Ala Asp Lys Asp Thr Leu Lys Ile
   945 950 955 960
   Val Thr Ala Tyr Thr Thr Asn Ser Thr Val Ala Thr Ala Thr Thr Asp
   965 970 975
   Ala Asn Gln Leu Gly Phe Ser Val Glu Pro Lys Gly Glu Gly Ser Thr
   980 985 990
   Thr Val Thr Val Val Val Thr Asp Gly Lys Asp Asn Ile Asn Val Ser
   995 1000 1005
   Phe Lys Val Asn Val Ser Thr Ala Ser Tyr Thr Ser Val Val Asn
   1010 1015 1020
   Ser Gly Asn Pro Val Lys Asp Phe Ala Pro Ala Val Pro Thr Ala
   1025 1030 1035
   Ala Thr Ala Lys Leu Pro Glu Phe Thr Ser Val Ala Lys Ala Gly
   1040 1045 1050
   Thr Ile Lys Val Gln Gly Phe Asp Ile Pro Leu Thr Leu Gly Ser
   1055 1060 1065
   Thr Gln Ala Gln Val Leu Glu Thr Leu Asn Asn Phe Ile Thr Asp
   1070 1075 1080
   Tyr Lys Ile Asn Ala Ser Ala Lys Leu Val Asp Lys Ala Ile Val
   1085 1090 1095
   Ile Ser Ser Thr Glu Thr Gly Asn Ala Ala Thr Leu Thr Val Glu
   1100 1105 1110
   Gly Asp Ser Thr Leu Glu Leu Val Ala Gly Thr Val Leu Gly Thr
   1115 1120 1125
   Val Ser Thr Thr Pro Gly Ser Val Gly Thr Ala Thr Pro Ala Lys
   1130 1135 1140
   Tyr Ala Val Lys Val Leu Asn Gly Val Ser Asn Gly Thr Lys Val
   1145 1150 1155
   Asp Phe Lys Phe Thr Ile Gly Ala Lys Thr Val Thr Val Thr Val
   1160 1165 1170
   Asp Ala Thr Ala Gly Ala Lys Ala Val Ser Asp Val Val Asp Thr
   1175 1180 1185
   Leu Ala Ala Ala Thr Leu Asp Gly Tyr Ser Ile Thr Lys Asn Gly
   1190 1195 1200
   Asp Val Ile Glu Leu Thr Gln Thr Thr Pro Ala Thr Thr Thr Asp
   1205 1210 1215
   Lys Leu Val Val Glu Thr Lys Lys Ala Asn
   1220 1225
<210> 3
   <211> 3300
   <212> DNA
   <213> Lysinibacillus sphaericus
<220>
   <221> CDS
   <222> (1)..(3300)
<400> 3
   atg gct aac caa cca aag aaa tac aaa aaa ttc gta gcg acg gca gca          48
   Met Ala Asn Gln Pro Lys Lys Tyr Lys Lys Phe Val Ala Thr Ala Ala
   1               5                   10                  15
   acg gca aca tta gta gca tct gct atc gta cca gtg gtt tct gcc gca      96
   Thr Ala Thr Leu Val Ala Ser Ala Ile Val Pro Val Val Ser Ala Ala
               20                  25                  30
   gga ttt tca gat gta gca ggt aac gac cac gaa gta gca atc aac gca     144
   Gly Phe Ser Asp Val Ala Gly Asn Asp His Glu Val Ala Ile Asn Ala
           35                  40                  45
   ctt gct gat gca ggt atc atc aat gga tac gct gac ggc aca ttc aaa     192
   Leu Ala Asp Ala Gly Ile Ile Asn Gly Tyr Ala Asp Gly Thr Phe Lys
       50                  55                  60
   cca aac caa aca att aac cgc ggt caa gtg gtt aaa tta tta ggt cgt     240
   Pro Asn Gln Thr Ile Asn Arg Gly Gln Val Val Lys Leu Leu Gly Arg
   65                  70                  75                  80
   tat tta gaa gca caa ggc caa gaa att cca gct gac tgg aat tca aaa     288
   Tyr Leu Glu Ala Gln Gly Gln Glu Ile Pro Ala Asp Trp Asn Ser Lys
                   85                  90                  95
   caa cgc ttc aac gat cta cca gta aca gct gaa gca gaa tta gta aaa     336
   Gln Arg Phe Asn Asp Leu Pro Val Thr Ala Glu Ala Glu Leu Val Lys
               100                 105                 110
   tat gct gca cta gca aaa gat gca ggc gta ttc aac ggt tca aac ggc     384
   Tyr Ala Ala Leu Ala Lys Asp Ala Gly Val Phe Asn Gly Ser Asn Gly
           115                 120                 125
   aac tta aac gct tca caa aca atg caa cgt caa caa atg gca gta gtt     432
   Asn Leu Asn Ala Ser Gln Thr Met Gln Arg Gln Gln Met Ala Val Val
       130                 135                 140
   tta gta cgt gct att aaa gaa atc gct ggc gta gac tta gta gct gag     480
   Leu Val Arg Ala Ile Lys Glu Ile Ala Gly Val Asp Leu Val Ala Glu
   145                 150                 155                 160
   tac aaa aaa gcg aat ttc gta aca gaa att ggt gac tta gac aaa gct     528
   Tyr Lys Lys Ala Asn Phe Val Thr Glu Ile Gly Asp Leu Asp Lys Ala
                   165                 170                 175
   tac tct gca gaa caa cgc aca gcg atc gtt gct tta gaa tat gcg ggt     576
   Tyr Ser Ala Glu Gln Arg Thr Ala Ile Val Ala Leu Glu Tyr Ala Gly
               180                 185                 190
   att aca aat gtt gct cac ttc aac cca ggt aac agc gta aca cgc gga     624
   Ile Thr Asn Val Ala His Phe Asn Pro Gly Asn Ser Val Thr Arg Gly
           195                 200                 205
   caa ttc gct tca ttc tta tac cgc aca atc gaa aac gtg gta aac gct     672
   Gln Phe Ala Ser Phe Leu Tyr Arg Thr Ile Glu Asn Val Val Asn Ala
       210                 215                 220
   cct gaa gct ggc gct gcg gca gtg aaa gct gta aac aac aca act gtt     720
   Pro Glu Ala Gly Ala Ala Ala Val Lys Ala Val Asn Asn Thr Thr Val
   225                 230                 235                 240
   gaa gta aca ttc gac gaa gaa gta gac aac gta caa gct ctt aac ttc     768
   Glu Val Thr Phe Asp Glu Glu Val Asp Asn Val Gln Ala Leu Asn Phe
                   245                 250                 255
   tta atc tct gat tta gaa gtg aaa aac gca gct gta aaa caa act aac     816
   Leu Ile Ser Asp Leu Glu Val Lys Asn Ala Ala Val Lys Gln Thr Asn
               260                 265                 270
   aaa aaa gtt gtt gtt tta act act gca cct caa act gct gac aaa gag     864
   Lys Lys Val Val Val Leu Thr Thr Ala Pro Gln Thr Ala Asp Lys Glu
           275                 280                 285
   tac act gta tct ctt ggc gaa gaa aaa att ggt act ttc aaa ggt atc      912
   Tyr Thr Val Ser Leu Gly Glu Glu Lys Ile Gly Thr Phe Lys Gly Ile
       290                 295                 300
   gca gct gta gtt cct act aaa gta gat tta gta gaa aaa tca gtt caa      960
   Ala Ala Val Val Pro Thr Lys Val Asp Leu Val Glu Lys Ser Val Gln
   305                 310                 315                 320
   ggt aaa ctt ggc caa caa gta aca ctg aaa gca caa gta aca gtt gct     1008
   Gly Lys Leu Gly Gln Gln Val Thr Leu Lys Ala Gln Val Thr Val Ala
                   325                 330                 335
   gaa ggt caa aca aaa gct ggt att cca gta act ttc ttc atc cca ggt     1056
   Glu Gly Gln Thr Lys Ala Gly Ile Pro Val Thr Phe Phe Ile Pro Gly
               340                 345                 350
   tct gct aat ggt gtg aaa tct cca gta aca gtt gaa gct gta aca gat     1104
   Ser Ala Asn Gly Val Lys Ser Pro Val Thr Val Glu Ala Val Thr Asp
           355                 360                 365
   gaa aac ggt gtt gct tct tat act tac act cgt tac gct gca aca aac     1152
   Glu Asn Gly Val Ala Ser Tyr Thr Tyr Thr Arg Tyr Ala Ala Thr Asn
       370                 375                 380
   gac aca gta act gta tac gcg aac ggt gac cgt tct aaa ttc tca act     1200
   Asp Thr Val Thr Val Tyr Ala Asn Gly Asp Arg Ser Lys Phe Ser Thr
   385                 390                 395                 400
   ggt tat gtc ttc tgg gct gtt gat caa cat cta acg att act gaa gta     1248
   Gly Tyr Val Phe Trp Ala Val Asp Gln His Leu Thr Ile Thr Glu Val
                   405                 410                 415
   aca act ggt aat aca atc aac aac gga gct aac aaa acg tat aaa gtc     1296
   Thr Thr Gly Asn Thr Ile Asn Asn Gly Ala Asn Lys Thr Tyr Lys Val
               420                 425                 430
   act tac aaa cac ccg gaa act ggt aga ccg gta tca ggt aaa gta cta     1344
   Thr Tyr Lys His Pro Glu Thr Gly Arg Pro Val Ser Gly Lys Val Leu
           435                 440                 445
   aac gtt tct gta ctt gaa aac att gat gta acc gtt gat aaa tta caa     1392
   Asn Val Ser Val Leu Glu Asn Ile Asp Val Thr Val Asp Lys Leu Gln
       450                 455                 460
   aac gtt acg gtg aat ggt gtt gcg gta gtt caa acg agc gaa aat aac     1440
   Asn Val Thr Val Asn Gly Val Ala Val Val Gln Thr Ser Glu Asn Asn
   465                 470                 475                 480
   atg cga gct gca caa atc aca act gat tca aaa ggt gaa gca aca ttc     1488
   Met Arg Ala Ala Gln Ile Thr Thr Asp Ser Lys Gly Glu Ala Thr Phe
                   485                 490                 495
   aca att tca ggt tct aac gct aca gtt act cca gta gta ttt gaa gct     1536
   Thr Ile Ser Gly Ser Asn Ala Thr Val Thr Pro Val Val Phe Glu Ala
               500                 505                 510
   gct tca act aat gtt gca aac act tac tct cca aaa tat aca gca gat     1584
   Ala Ser Thr Asn Val Ala Asn Thr Tyr Ser Pro Lys Tyr Thr Ala Asp
           515                 520                 525
   aca cta caa gct gtt gcg tct aaa ata aca ttt ggt gct att caa gct     1632
   Thr Leu Gln Ala Val Ala Ser Lys Ile Thr Phe Gly Ala Ile Gln Ala
       530                 535                 540
   gct tat aca att gaa gta act cgt gat ggt ggt gaa act gct gct act     1680
   Ala Tyr Thr Ile Glu Val Thr Arg Asp Gly Gly Glu Thr Ala Ala Thr
   545                 550                 555                 560
   gga gtt tta aac gga cgt aaa tac aat gta gtg gtt aaa gat aaa gat     1728
   Gly Val Leu Asn Gly Arg Lys Tyr Asn Val Val Val Lys Asp Lys Asp
                   565                 570                 575
   ggt aaa gta gct gct aac gaa att gtt aac gtg gca ttt aac gaa gat      1776
   Gly Lys Val Ala Ala Asn Glu Ile Val Asn Val Ala Phe Asn Glu Asp
               580                 585                 590
   att gat ggc gta att tct aca gtt act tct gct caa ttc gta aaa atc      1824
   Ile Asp Gly Val Ile Ser Thr Val Thr Ser Ala Gln Phe Val Lys Ile
           595                 600                 605
   gaa aac ggt aaa caa gtt ggc tac aat ggt aag aaa att acg gtt aca      1872
   Glu Asn Gly Lys Gln Val Gly Tyr Asn Gly Lys Lys Ile Thr Val Thr
       610                 615                 620
   aca aac tct aaa ggt gaa gca agc ttc gtc atc gga agt gac gct gta      1920
   Thr Asn Ser Lys Gly Glu Ala Ser Phe Val Ile Gly Ser Asp Ala Val
   625                 630                 635                 640
   aat tct tac gct aca cca atc gtt tgg att gac atc aat aac caa agt      1968
   Asn Ser Tyr Ala Thr Pro Ile Val Trp Ile Asp Ile Asn Asn Gln Ser
                   645                 650                 655
   ggt aaa gat gca aac tta gac aaa ggt gag cct tca gca att gca cca      2016
   Gly Lys Asp Ala Asn Leu Asp Lys Gly Glu Pro Ser Ala Ile Ala Pro
               660                 665                 670
   att tca tac ttc caa gca cca tat tta gat ggt tct aaa tta gta tct      2064
   Ile Ser Tyr Phe Gln Ala Pro Tyr Leu Asp Gly Ser Lys Leu Val Ser
           675                 680                 685
   tac aaa ggt aca act gaa act gac aaa ttt gtt agt gga gaa act gca      2112
   Tyr Lys Gly Thr Thr Glu Thr Asp Lys Phe Val Ser Gly Glu Thr Ala
       690                 695                 700
   aca ttt aaa gta caa ctt acg aac caa agt ggt aaa gtc gta cag aat      2160
   Thr Phe Lys Val Gln Leu Thr Asn Gln Ser Gly Lys Val Val Gln Asn
   705                 710                 715                 720
   tct agt tac aca aca aag gat gtt acg tac act gtt tac aac act ggt      2208
   Ser Ser Tyr Thr Thr Lys Asp Val Thr Tyr Thr Val Tyr Asn Thr Gly
                   725                 730                 735
   gct aat aat gta aaa gtt ggc gat gtt gaa atc gct cca aac cgc gtg      2256
   Ala Asn Asn Val Lys Val Gly Asp Val Glu Ile Ala Pro Asn Arg Val
               740                 745                 750
   cat aca tta gtt gcc gca aac ggt gaa atc gac gtt act act gta gat      2304
   His Thr Leu Val Ala Ala Asn Gly Glu Ile Asp Val Thr Thr Val Asp
           755                 760                 765
   ggt aaa tct tct tct gtg aaa gtt ctt gct act ggt gta gcg aaa gac      2352
   Gly Lys Ser Ser Ser Val Lys Val Leu Ala Thr Gly Val Ala Lys Asp
       770                 775                 780
   gga aac aaa gaa ttc gca ttt act tca aaa gaa gca act gca aca ttc      2400
   Gly Asn Lys Glu Phe Ala Phe Thr Ser Lys Glu Ala Thr Ala Thr Phe
   785                 790                 795                 800
   act tct tcg aaa gaa gta acg aac cta ttt act ggt gat att cat tca      2448
   Thr Ser Ser Lys Glu Val Thr Asn Leu Phe Thr Gly Asp Ile His Ser
                   805                 810                 815
   ttc gat aca gaa aaa gaa act atc gta ttt act gat ggt tcc gta cta      2496
   Phe Asp Thr Glu Lys Glu Thr Ile Val Phe Thr Asp Gly Ser Val Leu
               820                 825                 830
   aaa gat gcg gtg aaa tac gct ggt gaa aca gat aaa aaa tat gaa tat      2544
   Lys Asp Ala Val Lys Tyr Ala Gly Glu Thr Asp Lys Lys Tyr Glu Tyr
           835                     840                     845
   aga aat ata aat cgt gca cca att tct aaa gca gag ttt gta aat att        2592
   Arg Asn Ile Asn Arg Ala Pro Ile Ser Lys Ala Glu Phe Val Asn Ile
       850                     855                     860
   ctt aaa ggg gca att aca ggg gta aaa gta act tat gaa gtg aaa gat        2640
   Leu Lys Gly Ala Ile Thr Gly Val Lys Val Thr Tyr Glu Val Lys Asp
   865                     870                     875                     880
   aat acg gta ata ttc cat gtt ctt tct gta aat gct ggt ggt act aaa        2688
   Asn Thr Val Ile Phe His Val Leu Ser Val Asn Ala Gly Gly Thr Lys
                   885                     890                     895
   ccg gtt gat att att gga gat aaa ata tct gat gct act gct gtt aca        2736
   Pro Val Asp Ile Ile Gly Asp Lys Ile Ser Asp Ala Thr Ala Val Thr
               900                     905                     910
   acg gca aaa ggt aaa gtt aca gta aca cct aat att gct aca ctt tca        2784
   Thr Ala Lys Gly Lys Val Thr Val Thr Pro Asn Ile Ala Thr Leu Ser
           915                     920                     925
   act gat acg gcg gaa aat aaa cgt tta gct gta caa tca aat gtt gta        2832
   Thr Asp Thr Ala Glu Asn Lys Arg Leu Ala Val Gln Ser Asn Val Val
       930                     935                     940
   gct gaa att aac gac cca tca gta act gta agt gta gta gaa act tcg        2880
   Ala Glu Ile Asn Asp Pro Ser Val Thr Val Ser Val Val Glu Thr Ser
   945                     950                     955                     960
   aca gta gga act tat act gtg acg tta act aaa ggc gca agc act gct        2928
   Thr Val Gly Thr Tyr Thr Val Thr Leu Thr Lys Gly Ala Ser Thr Ala
                   965                     970                     975
   aca gta atg gtt aca cca aaa ttt ggt caa aaa tta act gct gaa tcg        2976
   Thr Val Met Val Thr Pro Lys Phe Gly Gln Lys Leu Thr Ala Glu Ser
               980                     985                     990
   att act tct tca ctt gca gtg aat  aca aat gga aaa gat  tta aat ctt      3024
   Ile Thr Ser Ser Leu Ala Val Asn  Thr Asn Gly Lys Asp  Leu Asn Leu
           995                     1000                    1005
   aat ctt  aca tct act ttt gct  ggt ttc aca atc gct  aat gta aat        3069
   Asn Leu  Thr Ser Thr Phe Ala  Gly Phe Thr Ile Ala  Asn Val Asn
       1010                     1015                     1020
   cca gat  aga gat aat tca tta  gac att act act tct  gtt aat aat        3114
   Pro Asp  Arg Asp Asn Ser Leu  Asp Ile Thr Thr Ser  Val Asn Asn
       1025                     1030                     1035
   aat gtg  att act ata tct aaa  tca cct tct gat tct  act gaa gct        3159
   Asn Val  Ile Thr Ile Ser Lys  Ser Pro Ser Asp Ser  Thr Glu Ala
       1040                     1045                     1050
   gca caa  tta atc gag aag act  tta aaa att tat ttc  gac gct caa        3204
   Ala Gln  Leu Ile Glu Lys Thr  Leu Lys Ile Tyr Phe  Asp Ala Gln
       1055                     1060                     1065
   gct act  ggt tat gcc aaa gaa  agc ttt gta gca act  att aca att        3249
   Ala Thr  Gly Tyr Ala Lys Glu  Ser Phe Val Ala Thr  Ile Thr Ile
       1070                     1075                     1080
   gat gat  gta acg aac cgt act  tta gct att aca gta  aag cca act        3294
   Asp Asp  Val Thr Asn Arg Thr  Leu Ala Ile Thr Val  Lys Pro Thr
       1085                     1090                     1095
   cca taa                                                                3300
   Pro
<210> 4
   <211> 1099
   <212> PRT
   <213> Lysinibacillus sphaericus
<400> 4
   Met Ala Asn Gln Pro Lys Lys Tyr Lys Lys Phe Val Ala Thr Ala Ala
   1               5                   10                  15
   Thr Ala Thr Leu Val Ala Ser Ala Ile Val Pro Val Val Ser Ala Ala
               20                  25                  30
   Gly Phe Ser Asp Val Ala Gly Asn Asp His Glu Val Ala Ile Asn Ala
           35                  40                  45
   Leu Ala Asp Ala Gly Ile Ile Asn Gly Tyr Ala Asp Gly Thr Phe Lys
       50                  55                  60
   Pro Asn Gln Thr Ile Asn Arg Gly Gln Val Val Lys Leu Leu Gly Arg
   65                  70                  75                  80
   Tyr Leu Glu Ala Gln Gly Gln Glu Ile Pro Ala Asp Trp Asn Ser Lys
                   85                  90                  95
   Gln Arg Phe Asn Asp Leu Pro Val Thr Ala Glu Ala Glu Leu Val Lys
               100                 105                 110
   Tyr Ala Ala Leu Ala Lys Asp Ala Gly Val Phe Asn Gly Ser Asn Gly
           115                 120                 125
   Asn Leu Asn Ala Ser Gln Thr Met Gln Arg Gln Gln Met Ala Val Val
       130                 135                 140
   Leu Val Arg Ala Ile Lys Glu Ile Ala Gly Val Asp Leu Val Ala Glu
   145                 150                 155                 160
   Tyr Lys Lys Ala Asn Phe Val Thr Glu Ile Gly Asp Leu Asp Lys Ala
                   165                 170                 175
   Tyr Ser Ala Glu Gln Arg Thr Ala Ile Val Ala Leu Glu Tyr Ala Gly
               180                 185                 190
   Ile Thr Asn Val Ala His Phe Asn Pro Gly Asn Ser Val Thr Arg Gly
           195                 200                 205
   Gln Phe Ala Ser Phe Leu Tyr Arg Thr Ile Glu Asn Val Val Asn Ala
       210                 215                 220
   Pro Glu Ala Gly Ala Ala Ala Val Lys Ala Val Asn Asn Thr Thr Val
   225                 230                 235                 240
   Glu Val Thr Phe Asp Glu Glu Val Asp Asn Val Gln Ala Leu Asn Phe
                   245                 250                 255
   Leu Ile Ser Asp Leu Glu Val Lys Asn Ala Ala Val Lys Gln Thr Asn
               260                 265                 270
   Lys Lys Val Val Val Leu Thr Thr Ala Pro Gln Thr Ala Asp Lys Glu
           275                 280                 285
   Tyr Thr Val Ser Leu Gly Glu Glu Lys Ile Gly Thr Phe Lys Gly Ile
       290                 295                 300
   Ala Ala Val Val Pro Thr Lys Val Asp Leu Val Glu Lys Ser Val Gln
   305                 310                 315                 320
   Gly Lys Leu Gly Gln Gln Val Thr Leu Lys Ala Gln Val Thr Val Ala
                   325                 330                 335
   Glu Gly Gln Thr Lys Ala Gly Ile Pro Val Thr Phe Phe Ile Pro Gly
               340                 345                 350
   Ser Ala Asn Gly Val Lys Ser Pro Val Thr Val Glu Ala Val Thr Asp
           355                 360                 365
   Glu Asn Gly Val Ala Ser Tyr Thr Tyr Thr Arg Tyr Ala Ala Thr Asn
       370                 375                 380
   Asp Thr Val Thr Val Tyr Ala Asn Gly Asp Arg Ser Lys Phe Ser Thr
   385                 390                 395                 400
   Gly Tyr Val Phe Trp Ala Val Asp Gln His Leu Thr Ile Thr Glu Val
                   405                 410                 415
   Thr Thr Gly Asn Thr Ile Asn Asn Gly Ala Asn Lys Thr Tyr Lys Val
               420                 425                 430
   Thr Tyr Lys His Pro Glu Thr Gly Arg Pro Val Ser Gly Lys Val Leu
           435                 440                 445
   Asn Val Ser Val Leu Glu Asn Ile Asp Val Thr Val Asp Lys Leu Gln
       450                 455                 460
   Asn Val Thr Val Asn Gly Val Ala Val Val Gln Thr Ser Glu Asn Asn
   465                 470                 475                 480
   Met Arg Ala Ala Gln Ile Thr Thr Asp Ser Lys Gly Glu Ala Thr Phe
                   485                 490                 495
   Thr Ile Ser Gly Ser Asn Ala Thr Val Thr Pro Val Val Phe Glu Ala
               500                 505                 510
   Ala Ser Thr Asn Val Ala Asn Thr Tyr Ser Pro Lys Tyr Thr Ala Asp
   515 520 525
   Thr Leu Gln Ala Val Ala Ser Lys Ile Thr Phe Gly Ala Ile Gln Ala
   530 535 540
   Ala Tyr Thr Ile Glu Val Thr Arg Asp Gly Gly Glu Thr Ala Ala Thr
   545 550 555 560
   Gly Val Leu Asn Gly Arg Lys Tyr Asn Val Val Val Lys Asp Lys Asp
   565 570 575
   Gly Lys Val Ala Ala Asn Glu Ile Val Asn Val Ala Phe Asn Glu Asp
   580 585 590
   Ile Asp Gly Val Ile Ser Thr Val Thr Ser Ala Gln Phe Val Lys Ile
   595 600 605
   Glu Asn Gly Lys Gln Val Gly Tyr Asn Gly Lys Lys Ile Thr Val Thr
   610 615 620
   Thr Asn Ser Lys Gly Glu Ala Ser Phe Val Ile Gly Ser Asp Ala Val
   625 630 635 640
   Asn Ser Tyr Ala Thr Pro Ile Val Trp Ile Asp Ile Asn Asn Gln Ser
   645 650 655
   Gly Lys Asp Ala Asn Leu Asp Lys Gly Glu Pro Ser Ala Ile Ala Pro
   660 665 670
   Ile Ser Tyr Phe Gln Ala Pro Tyr Leu Asp Gly Ser Lys Leu Val Ser
   675 680 685
   Tyr Lys Gly Thr Thr Glu Thr Asp Lys Phe Val Ser Gly Glu Thr Ala
   690 695 700
   Thr Phe Lys Val Gln Leu Thr Asn Gln Ser Gly Lys Val Val Gln Asn
   705 710 715 720
   Ser Ser Tyr Thr Thr Lys Asp Val Thr Tyr Thr Val Tyr Asn Thr Gly
   725 730 735
   Ala Asn Asn Val Lys Val Gly Asp Val Glu Ile Ala Pro Asn Arg Val
   740 745 750
   His Thr Leu Val Ala Ala Asn Gly Glu Ile Asp Val Thr Thr Val Asp
   755 760 765
   Gly Lys Ser Ser Ser Val Lys Val Leu Ala Thr Gly Val Ala Lys Asp
   770 775 780
   Gly Asn Lys Glu Phe Ala Phe Thr Ser Lys Glu Ala Thr Ala Thr Phe
   785 790 795 800
   Thr Ser Ser Lys Glu Val Thr Asn Leu Phe Thr Gly Asp Ile His Ser
   805 810 815
   Phe Asp Thr Glu Lys Glu Thr Ile Val Phe Thr Asp Gly Ser Val Leu
   820 825 830
   Lys Asp Ala Val Lys Tyr Ala Gly Glu Thr Asp Lys Lys Tyr Glu Tyr
   835 840 845
   Arg Asn Ile Asn Arg Ala Pro Ile Ser Lys Ala Glu Phe Val Asn Ile
   850 855 860
   Leu Lys Gly Ala Ile Thr Gly Val Lys Val Thr Tyr Glu Val Lys Asp
   865 870 875 880
   Asn Thr Val Ile Phe His Val Leu Ser Val Asn Ala Gly Gly Thr Lys
   885 890 895
   Pro Val Asp Ile Ile Gly Asp Lys Ile Ser Asp Ala Thr Ala Val Thr
   900 905 910
   Thr Ala Lys Gly Lys Val Thr Val Thr Pro Asn Ile Ala Thr Leu Ser
   915 920 925
   Thr Asp Thr Ala Glu Asn Lys Arg Leu Ala Val Gln Ser Asn Val Val
   930 935 940
   Ala Glu Ile Asn Asp Pro Ser Val Thr Val Ser Val Val Glu Thr Ser
   945 950 955 960
   Thr Val Gly Thr Tyr Thr Val Thr Leu Thr Lys Gly Ala Ser Thr Ala
   965 970 975
   Thr Val Met Val Thr Pro Lys Phe Gly Gln Lys Leu Thr Ala Glu Ser
   980 985 990
   Ile Thr Ser Ser Leu Ala Val Asn Thr Asn Gly Lys Asp Leu Asn Leu
   995 1000 1005
   Asn Leu Thr Ser Thr Phe Ala Gly Phe Thr Ile Ala Asn Val Asn
   1010 1015 1020
   Pro Asp Arg Asp Asn Ser Leu Asp Ile Thr Thr Ser Val Asn Asn
   1025 1030 1035
   Asn Val Ile Thr Ile Ser Lys Ser Pro Ser Asp Ser Thr Glu Ala
   1040 1045 1050
   Ala Gln Leu Ile Glu Lys Thr Leu Lys Ile Tyr Phe Asp Ala Gln
   1055 1060 1065
   Ala Thr Gly Tyr Ala Lys Glu Ser Phe Val Ala Thr Ile Thr Ile
       1070                1075                1080
   Asp Asp Val Thr Asn Arg Thr Leu Ala Ile Thr Val Lys Pro Thr
       1085                1090                1095
   Pro
<210> 5
   <211> 3714
   <212> DNA
   <213> Lysinibacillus sphaericus
<220>
   <221> CDS
   <222> (1)..(3714)
<400> 5
   atg gct aac caa cca aag aaa tac aaa aaa ttc gta gct acg gct gca       48
   Met Ala Asn Gln Pro Lys Lys Tyr Lys Lys Phe Val Ala Thr Ala Ala
   1               5                   10                  15
   acg gct act tta gta gct tct gct atc gta cca gtg gct tct gca gca       96
   Thr Ala Thr Leu Val Ala Ser Ala Ile Val Pro Val Ala Ser Ala Ala
               20                  25                  30
   gga ttc tca gat gta gca ggt aac gac cac gaa gta gca atc aac gca      144
   Gly Phe Ser Asp Val Ala Gly Asn Asp His Glu Val Ala Ile Asn Ala
           35                  40                  45
   ctt gct gat gca ggt atc atc aat gga tac gct gac ggc tca ttc aaa      192
   Leu Ala Asp Ala Gly Ile Ile Asn Gly Tyr Ala Asp Gly Ser Phe Lys
       50                  55                  60
   cca aac caa aca att aac cgc ggt caa gtg gtt aaa tta tta ggt cgt      240
   Pro Asn Gln Thr Ile Asn Arg Gly Gln Val Val Lys Leu Leu Gly Arg
   65                  70                  75                  80
   tat tta gaa gca caa ggc caa gaa att cca gct gac tgg aat tca aaa      288
   Tyr Leu Glu Ala Gln Gly Gln Glu Ile Pro Ala Asp Trp Asn Ser Lys
                   85                  90                  95
   caa cgc ttc aac gat cta cca gta aca gct gaa gaa gaa tta gtt aaa      336
   Gln Arg Phe Asn Asp Leu Pro Val Thr Ala Glu Glu Glu Leu Val Lys
               100                 105                 110
   tat gct gca ctt gca aaa gat gca ggc gta ttc aac ggt tca aat ggc      384
   Tyr Ala Ala Leu Ala Lys Asp Ala Gly Val Phe Asn Gly Ser Asn Gly
           115                 120                 125
   aac cta aac gct tca caa aca atg caa cgt caa caa atg gca gta gtt      432
   Asn Leu Asn Ala Ser Gln Thr Met Gln Arg Gln Gln Met Ala Val Val
       130                 135                 140
   tta gta cgt gct att aaa gaa atc gca ggc gta gac tta gta gct gag      480
   Leu Val Arg Ala Ile Lys Glu Ile Ala Gly Val Asp Leu Val Ala Glu
   145                 150                 155                 160
   tac aaa aaa gcg aac ttc gta aca gaa att ggt gac tta gac aaa gct      528
   Tyr Lys Lys Ala Asn Phe Val Thr Glu Ile Gly Asp Leu Asp Lys Ala
                   165                 170                 175
   tac tct gca gaa caa cgc aca gcg atc gtt gct tta gaa tat gcg ggt      576
   Tyr Ser Ala Glu Gln Arg Thr Ala Ile Val Ala Leu Glu Tyr Ala Gly
               180                 185                 190
   att aca aat gtt gct cac ttc aac cca ggt aac agc gta aca cgc gga     624
   Ile Thr Asn Val Ala His Phe Asn Pro Gly Asn Ser Val Thr Arg Gly
           195                 200                 205
   caa ttc gct tca ttc tta tac cgc aca atc gaa aac gtt gta aac aac     672
   Gln Phe Ala Ser Phe Leu Tyr Arg Thr Ile Glu Asn Val Val Asn Asn
       210                 215                 220
   cca gaa gct ggt gtt gcg gca gta aaa gct atc aac aac aca act gtt     720
   Pro Glu Ala Gly Val Ala Ala Val Lys Ala Ile Asn Asn Thr Thr Val
   225                 230                 235                 240
   gaa gta aca ttc gac gaa gaa gta gac aac gta caa gct ctt aac ttc     768
   Glu Val Thr Phe Asp Glu Glu Val Asp Asn Val Gln Ala Leu Asn Phe
                   245                 250                 255
   tta atc tct gat ctt gaa gtg aaa aac gca gct gtt aaa tta aca aac     816
   Leu Ile Ser Asp Leu Glu Val Lys Asn Ala Ala Val Lys Leu Thr Asn
               260                 265                 270
   aaa aaa gtt gtt gta tta aca act gct gct caa aca gct gac aaa gag     864
   Lys Lys Val Val Val Leu Thr Thr Ala Ala Gln Thr Ala Asp Lys Glu
           275                 280                 285
   tac act gta tct ctt ggc gaa gaa aaa atc ggt aca ttc aaa ggt atc     912
   Tyr Thr Val Ser Leu Gly Glu Glu Lys Ile Gly Thr Phe Lys Gly Ile
       290                 295                 300
   gca gct gta gtt cct act aaa gta gat tta gta gaa aaa tca gtt caa     960
   Ala Ala Val Val Pro Thr Lys Val Asp Leu Val Glu Lys Ser Val Gln
   305                 310                 315                 320
   ggt aaa ctt ggc caa caa gta aca ctt aaa gca caa gta act gtt gct    1008
   Gly Lys Leu Gly Gln Gln Val Thr Leu Lys Ala Gln Val Thr Val Ala
                   325                 330                 335
   gaa ggt caa aca aaa gct ggt att cca gta act ttc ttc atc cca ggt    1056
   Glu Gly Gln Thr Lys Ala Gly Ile Pro Val Thr Phe Phe Ile Pro Gly
               340                 345                 350
   tct gct aat ggc gtg aaa act cca gta aca gtt gaa gct gta aca aac    1104
   Ser Ala Asn Gly Val Lys Thr Pro Val Thr Val Glu Ala Val Thr Asn
           355                 360                 365
   gaa gaa ggt atc gca act tac tct tac act cgt tat gct gca aca aac    1152
   Glu Glu Gly Ile Ala Thr Tyr Ser Tyr Thr Arg Tyr Ala Ala Thr Asn
       370                 375                 380
   gac act gta act gta tat gca aac ggt gac cgt tct aaa ttc tca act    1200
   Asp Thr Val Thr Val Tyr Ala Asn Gly Asp Arg Ser Lys Phe Ser Thr
   385                 390                 395                 400
   ggt tat gta ttc tgg gca gtt gac caa act tta gaa atc act gaa gta    1248
   Gly Tyr Val Phe Trp Ala Val Asp Gln Thr Leu Glu Ile Thr Glu Val
                   405                 410                 415
   aca act ggt gct act atc aac aac ggc gct aac aaa act tac aaa gtt    1296
   Thr Thr Gly Ala Thr Ile Asn Asn Gly Ala Asn Lys Thr Tyr Lys Val
               420                 425                 430
   act tac aaa cac cca gaa act ggt aaa cca gta tca ggt aaa gta ctt    1344
   Thr Tyr Lys His Pro Glu Thr Gly Lys Pro Val Ser Gly Lys Val Leu
           435                 440                 445
   aac gtt tca gta aaa gaa aac att gac gta act gtt gat aaa tta caa    1392
   Asn Val Ser Val Lys Glu Asn Ile Asp Val Thr Val Asp Lys Leu Gln
           450                 455                 460
   aac gtt acg gtg aat ggt att gct gta gtt caa aca agc gat aac aac      1440
   Asn Val Thr Val Asn Gly Ile Ala Val Val Gln Thr Ser Asp Asn Asn
   465                 470                 475                 480
   aca cgt gct gct caa atc aca act gac tct aaa ggt gaa gca aca ttc      1488
   Thr Arg Ala Ala Gln Ile Thr Thr Asp Ser Lys Gly Glu Ala Thr Phe
                   485                 490                 495
   aca gtt tca ggt tct aac gct gaa gta act cca gtg gta ttt gaa gca      1536
   Thr Val Ser Gly Ser Asn Ala Glu Val Thr Pro Val Val Phe Glu Ala
               500                 505                 510
   act cca gta caa gtt act aac aca aat ggt gca gta aca aca act agc      1584
   Thr Pro Val Gln Val Thr Asn Thr Asn Gly Ala Val Thr Thr Thr Ser
           515                 520                 525
   tac act caa aaa tac act gct gat att tta caa gct tct gct gcg aaa      1632
   Tyr Thr Gln Lys Tyr Thr Ala Asp Ile Leu Gln Ala Ser Ala Ala Lys
       530                 535                 540
   gta aca ttt ggt gct gtt caa gct gct tac act ctt gaa gta act cgc      1680
   Val Thr Phe Gly Ala Val Gln Ala Ala Tyr Thr Leu Glu Val Thr Arg
   545                 550                 555                 560
   gat ggt ggc gaa gtt gcg gca act ggt gtt gaa aac ggc cgt aaa tat      1728
   Asp Gly Gly Glu Val Ala Ala Thr Gly Val Glu Asn Gly Arg Lys Tyr
                   565                 570                 575
   aac tta gta gta aaa gat aaa gat ggt aaa tta gct gct aac gaa aca      1776
   Asn Leu Val Val Lys Asp Lys Asp Gly Lys Leu Ala Ala Asn Glu Thr
               580                 585                 590
   gta aac gtt gca ttt aac gaa gat att gac gga gta att tct aca gtt      1824
   Val Asn Val Ala Phe Asn Glu Asp Ile Asp Gly Val Ile Ser Thr Val
           595                 600                 605
   act tct gca gaa ttc gta aaa gta gaa aac gat aaa caa gtt cgc tac      1872
   Thr Ser Ala Glu Phe Val Lys Val Glu Asn Asp Lys Gln Val Arg Tyr
       610                 615                 620
   gaa ggc aaa aaa atc act gtt aaa aca aat tct aaa ggt gaa gca agc      1920
   Glu Gly Lys Lys Ile Thr Val Lys Thr Asn Ser Lys Gly Glu Ala Ser
   625                 630                 635                 640
   ttc gta att tca agt gaa gct gta aac act tat gct aca cca atc gct      1968
   Phe Val Ile Ser Ser Glu Ala Val Asn Thr Tyr Ala Thr Pro Ile Ala
                   645                 650                 655
   tgg atc gat atc aac aac caa agc gct aaa gat gca aac tta gac aaa      2016
   Trp Ile Asp Ile Asn Asn Gln Ser Ala Lys Asp Ala Asn Leu Asp Lys
               660                 665                 670
   ggt gaa cca tct gca gtt gca cca att tca tac ttc caa gct gaa tat      2064
   Gly Glu Pro Ser Ala Val Ala Pro Ile Ser Tyr Phe Gln Ala Glu Tyr
           675                 680                 685
   tta gat ggt tct aaa tta gta tct tac aaa gat aca act gaa act gat      2112
   Leu Asp Gly Ser Lys Leu Val Ser Tyr Lys Asp Thr Thr Glu Thr Asp
       690                 695                 700
   aaa ttc act ggt agc gaa act gca aca ttc aaa gta caa ctt aca aac      2160
   Lys Phe Thr Gly Ser Glu Thr Ala Thr Phe Lys Val Gln Leu Thr Asn
   705                 710                 715                 720
   caa agt ggt aaa gta gtt aaa aac tct ggt tat aca aca cca aat gtt      2208
   Gln Ser Gly Lys Val Val Lys Asn Ser Gly Tyr Thr Thr Pro Asn Val
                   725                 730                 735
   tct tac act gta tac aac act ggt gca aac aat gta aaa gtt gac ggt      2256
   Ser Tyr Thr Val Tyr Asn Thr Gly Ala Asn Asn Val Lys Val Asp Gly
               740                 745                 750
   gta gaa atc gca cca aac cgc gtg cat aca gta gtt gca aaa gac ggt      2304
   Val Glu Ile Ala Pro Asn Arg Val His Thr Val Val Ala Lys Asp Gly
           755                 760                 765
   gta atc aat gtt act aca gta gat aat aag tct tct tct gtt aaa gtt      2352
   Val Ile Asn Val Thr Thr Val Asp Asn Lys Ser Ser Ser Val Lys Val
       770                 775                 780
   ctt gct act ggt gta gct aaa caa aca aac ggt aac aaa gaa ttt gca      2400
   Leu Ala Thr Gly Val Ala Lys Gln Thr Asn Gly Asn Lys Glu Phe Ala
   785                 790                 795                 800
   ttc act tct aaa gaa gca act gca aca ttc aca gct act aat gaa gta      2448
   Phe Thr Ser Lys Glu Ala Thr Ala Thr Phe Thr Ala Thr Asn Glu Val
                   805                 810                 815
   tct aat cca tat act ggt gct gtt aaa cat tac aat aca gac aag aaa      2496
   Ser Asn Pro Tyr Thr Gly Ala Val Lys His Tyr Asn Thr Asp Lys Lys
               820                 825                 830
   act att aca ttc gat aat aaa gat gca att aaa tat gcg ggt gta aaa      2544
   Thr Ile Thr Phe Asp Asn Lys Asp Ala Ile Lys Tyr Ala Gly Val Lys
           835                 840                 845
   ggt aaa act tac aaa tac ttt ggt ctt ggt agc act cca att gca aat      2592
   Gly Lys Thr Tyr Lys Tyr Phe Gly Leu Gly Ser Thr Pro Ile Ala Asn
       850                 855                 860
   gca gat gca ttt atc gca aca tta agt ggt caa ggt gct ggt aac caa      2640
   Ala Asp Ala Phe Ile Ala Thr Leu Ser Gly Gln Gly Ala Gly Asn Gln
   865                 870                 875                 880
   gtt act gta act tat aaa gaa gaa gat gat gtt gta tca ttc tac att      2688
   Val Thr Val Thr Tyr Lys Glu Glu Asp Asp Val Val Ser Phe Tyr Ile
                   885                 890                 895
   att agt gtt gta aat ggt ggt att gga aat cct aca act gac cct gct      2736
   Ile Ser Val Val Asn Gly Gly Ile Gly Asn Pro Thr Thr Asp Pro Ala
               900                 905                 910
   cta gct aac ggt gta aca gga aca att gcg ttc acg aat aca tca ttc      2784
   Leu Ala Asn Gly Val Thr Gly Thr Ile Ala Phe Thr Asn Thr Ser Phe
           915                 920                 925
   aat gca agt gca aat caa aca att aca gtt aaa gat gct gat tta aat      2832
   Asn Ala Ser Ala Asn Gln Thr Ile Thr Val Lys Asp Ala Asp Leu Asn
       930                 935                 940
   gta aat gct act gta gct gat act aca act gtt aca att aca gat tct      2880
   Val Asn Ala Thr Val Ala Asp Thr Thr Thr Val Thr Ile Thr Asp Ser
   945                 950                 955                 960
   gct aaa act aca ttc aac atc act tta act gaa act ggg gtt aat act      2928
   Ala Lys Thr Thr Phe Asn Ile Thr Leu Thr Glu Thr Gly Val Asn Thr
                   965                 970                 975
   gga gaa ttc aca gga act ctt act tca gct caa cta gct tta tta aaa      2976
   Gly Glu Phe Thr Gly Thr Leu Thr Ser Ala Gln Leu Ala Leu Leu Lys
               980                 985                 990
   gat ggt gtg att aca gca act tat  aat gat gct aaa aat  gct aac aat      3024
   Asp Gly Val Ile Thr Ala Thr Tyr  Asn Asp Ala Lys Asn  Ala Asn Asn
           995                 1000                1005
   gtt gca gct act gct act gct act gct aca tta aat aca aca gtt        3069
   Val Ala Ala Thr Ala Thr Ala Thr Ala Thr Leu Asn Thr Thr Val
       1010                1015                1020
   ggc gct gta aac ttt gct gct aaa gca act act gaa tat tca gaa        3114
   Gly Ala Val Asn Phe Ala Ala Lys Ala Thr Thr Glu Tyr Ser Glu
       1025                1030                1035
   ggt gtt ggt aca gct gct aaa gtt gaa ggt agt act gta tta act        3159
   Gly Val Gly Thr Ala Ala Lys Val Glu Gly Ser Thr Val Leu Thr
       1040                1045                1050
   tca aaa gtt gat act ggt gat ctt gta tta gta gta gat ggt gtt        3204
   Ser Lys Val Asp Thr Gly Asp Leu Val Leu Val Val Asp Gly Val
       1055                1060                1065
   caa ttc aca act gtt gtt act gca att gat cct ggt act tca gct        3249
   Gln Phe Thr Thr Val Val Thr Ala Ile Asp Pro Gly Thr Ser Ala
       1070                1075                1080
   aca gct agt tta act gca gta gtt gct gaa atc aat gca gca gct        3294
   Thr Ala Ser Leu Thr Ala Val Val Ala Glu Ile Asn Ala Ala Ala
       1085                1090                1095
   aaa aaa gtt gga ttc aca gct gat gtt gca act gtt gat gct gat        3339
   Lys Lys Val Gly Phe Thr Ala Asp Val Ala Thr Val Asp Ala Asp
       1100                1105                1110
   aaa atc gtt ctt aca agc cca aca aaa ggt aca tct tca tct gta        3384
   Lys Ile Val Leu Thr Ser Pro Thr Lys Gly Thr Ser Ser Ser Val
       1115                1120                1125
   gaa gtt aaa gat tta gga aca act act gga tta ggt tta act aaa        3429
   Glu Val Lys Asp Leu Gly Thr Thr Thr Gly Leu Gly Leu Thr Lys
       1130                1135                1140
   gca cct gaa gta aaa ggt act gct ggt gct gct gta gca act caa        3474
   Ala Pro Glu Val Lys Gly Thr Ala Gly Ala Ala Val Ala Thr Gln
       1145                1150                1155
   tgg aag ttc aca gta act act act cca gct gtt ggt gaa act att        3519
   Trp Lys Phe Thr Val Thr Thr Thr Pro Ala Val Gly Glu Thr Ile
       1160                1165                1170
   aca gtt aaa gtt gga act aaa gaa gct tct cac aaa gtt gtt gca        3564
   Thr Val Lys Val Gly Thr Lys Glu Ala Ser His Lys Val Val Ala
       1175                1180                1185
   ggt aac gat tta gct gct att gca act gct tta aat act gct atc        3609
   Gly Asn Asp Leu Ala Ala Ile Ala Thr Ala Leu Asn Thr Ala Ile
       1190                1195                1200
   ggt gct gac tat aat atc gca ctt gtt act ggt tct aat gta att        3654
   Gly Ala Asp Tyr Asn Ile Ala Leu Val Thr Gly Ser Asn Val Ile
       1205                1210                1215
   aca gtt aca caa gct act cca gct aaa aca act gat gaa tta tca        3699
   Thr Val Thr Gln Ala Thr Pro Ala Lys Thr Thr Asp Glu Leu Ser
       1220                1225                1230
   gtt act gtt aca aaa                                                3714
   Val Thr Val Thr Lys
       1235
<210> 6
   <211> 1238
   <212> PRT
   <213> Lysinibacillus sphaericus
<400> 6
   Met Ala Asn Gln Pro Lys Lys Tyr Lys Lys Phe Val Ala Thr Ala Ala
   1               5                   10                  15
   Thr Ala Thr Leu Val Ala Ser Ala Ile Val Pro Val Ala Ser Ala Ala
               20                  25                  30
   Gly Phe Ser Asp Val Ala Gly Asn Asp His Glu Val Ala Ile Asn Ala
           35                  40                  45
   Leu Ala Asp Ala Gly Ile Ile Asn Gly Tyr Ala Asp Gly Ser Phe Lys
       50                  55                  60
   Pro Asn Gln Thr Ile Asn Arg Gly Gln Val Val Lys Leu Leu Gly Arg
   65                  70                  75                  80
   Tyr Leu Glu Ala Gln Gly Gln Glu Ile Pro Ala Asp Trp Asn Ser Lys
                   85                  90                  95
   Gln Arg Phe Asn Asp Leu Pro Val Thr Ala Glu Glu Glu Leu Val Lys
               100                 105                 110
   Tyr Ala Ala Leu Ala Lys Asp Ala Gly Val Phe Asn Gly Ser Asn Gly
           115                 120                 125
   Asn Leu Asn Ala Ser Gln Thr Met Gln Arg Gln Gln Met Ala Val Val
       130                 135                 140
   Leu Val Arg Ala Ile Lys Glu Ile Ala Gly Val Asp Leu Val Ala Glu
   145                 150                 155                 160
   Tyr Lys Lys Ala Asn Phe Val Thr Glu Ile Gly Asp Leu Asp Lys Ala
                   165                 170                 175
   Tyr Ser Ala Glu Gln Arg Thr Ala Ile Val Ala Leu Glu Tyr Ala Gly
               180                 185                 190
   Ile Thr Asn Val Ala His Phe Asn Pro Gly Asn Ser Val Thr Arg Gly
           195                 200                 205
   Gln Phe Ala Ser Phe Leu Tyr Arg Thr Ile Glu Asn Val Val Asn Asn
       210                 215                 220
   Pro Glu Ala Gly Val Ala Ala Val Lys Ala Ile Asn Asn Thr Thr Val
   225                 230                 235                 240
   Glu Val Thr Phe Asp Glu Glu Val Asp Asn Val Gln Ala Leu Asn Phe
                   245                 250                 255
   Leu Ile Ser Asp Leu Glu Val Lys Asn Ala Ala Val Lys Leu Thr Asn
               260                 265                 270
   Lys Lys Val Val Val Leu Thr Thr Ala Ala Gln Thr Ala Asp Lys Glu
           275                     280                     285
   Tyr Thr Val Ser Leu Gly Glu Glu Lys Ile Gly Thr Phe Lys Gly Ile
       290                     295                     300
   Ala Ala Val Val Pro Thr Lys Val Asp Leu Val Glu Lys Ser Val Gln
   305                     310                     315                     320
   Gly Lys Leu Gly Gln Gln Val Thr Leu Lys Ala Gln Val Thr Val Ala
                   325                     330                     335
   Glu Gly Gln Thr Lys Ala Gly Ile Pro Val Thr Phe Phe Ile Pro Gly
               340                     345                     350
   Ser Ala Asn Gly Val Lys Thr Pro Val Thr Val Glu Ala Val Thr Asn
           355                     360                     365
   Glu Glu Gly Ile Ala Thr Tyr Ser Tyr Thr Arg Tyr Ala Ala Thr Asn
       370                     375                     380
   Asp Thr Val Thr Val Tyr Ala Asn Gly Asp Arg Ser Lys Phe Ser Thr
   385                     390                     395                     400
   Gly Tyr Val Phe Trp Ala Val Asp Gln Thr Leu Glu Ile Thr Glu Val
                   405                     410                     415
   Thr Thr Gly Ala Thr Ile Asn Asn Gly Ala Asn Lys Thr Tyr Lys Val
               420                     425                     430
   Thr Tyr Lys His Pro Glu Thr Gly Lys Pro Val Ser Gly Lys Val Leu
           435                     440                     445
   Asn Val Ser Val Lys Glu Asn Ile Asp Val Thr Val Asp Lys Leu Gln
       450                     455                     460
   Asn Val Thr Val Asn Gly Ile Ala Val Val Gln Thr Ser Asp Asn Asn
   465                     470                     475                     480
   Thr Arg Ala Ala Gln Ile Thr Thr Asp Ser Lys Gly Glu Ala Thr Phe
                   485                     490                     495
   Thr Val Ser Gly Ser Asn Ala Glu Val Thr Pro Val Val Phe Glu Ala
               500                     505                     510
   Thr Pro Val Gln Val Thr Asn Thr Asn Gly Ala Val Thr Thr Thr Ser
           515                     520                     525
   Tyr Thr Gln Lys Tyr Thr Ala Asp Ile Leu Gln Ala Ser Ala Ala Lys
       530                     535                     540
   Val Thr Phe Gly Ala Val Gln Ala Ala Tyr Thr Leu Glu Val Thr Arg
   545 550 555 560
   Asp Gly Gly Glu Val Ala Ala Thr Gly Val Glu Asn Gly Arg Lys Tyr
   565 570 575
   Asn Leu Val Val Lys Asp Lys Asp Gly Lys Leu Ala Ala Asn Glu Thr
   580 585 590
   Val Asn Val Ala Phe Asn Glu Asp Ile Asp Gly Val Ile Ser Thr Val
   595 600 605
   Thr Ser Ala Glu Phe Val Lys Val Glu Asn Asp Lys Gln Val Arg Tyr
   610 615 620
   Glu Gly Lys Lys Ile Thr Val Lys Thr Asn Ser Lys Gly Glu Ala Ser
   625 630 635 640
   Phe Val Ile Ser Ser Glu Ala Val Asn Thr Tyr Ala Thr Pro Ile Ala
   645 650 655
   Trp Ile Asp Ile Asn Asn Gln Ser Ala Lys Asp Ala Asn Leu Asp Lys
   660 665 670
   Gly Glu Pro Ser Ala Val Ala Pro Ile Ser Tyr Phe Gln Ala Glu Tyr
   675 680 685
   Leu Asp Gly Ser Lys Leu Val Ser Tyr Lys Asp Thr Thr Glu Thr Asp
   690 695 700
   Lys Phe Thr Gly Ser Glu Thr Ala Thr Phe Lys Val Gln Leu Thr Asn
   705 710 715 720
   Gln Ser Gly Lys Val Val Lys Asn Ser Gly Tyr Thr Thr Pro Asn Val
   725 730 735
   Ser Tyr Thr Val Tyr Asn Thr Gly Ala Asn Asn Val Lys Val Asp Gly
   740 745 750
   Val Glu Ile Ala Pro Asn Arg Val His Thr Val Val Ala Lys Asp Gly
   755 760 765
   Val Ile Asn Val Thr Thr Val Asp Asn Lys Ser Ser Ser Val Lys Val
   770 775 780
   Leu Ala Thr Gly Val Ala Lys Gln Thr Asn Gly Asn Lys Glu Phe Ala
   785 790 795 800
   Phe Thr Ser Lys Glu Ala Thr Ala Thr Phe Thr Ala Thr Asn Glu Val
   805 810 815
   Ser Asn Pro Tyr Thr Gly Ala Val Lys His Tyr Asn Thr Asp Lys Lys
               820                 825                 830
   Thr Ile Thr Phe Asp Asn Lys Asp Ala Ile Lys Tyr Ala Gly Val Lys
           835                 840                 845
   Gly Lys Thr Tyr Lys Tyr Phe Gly Leu Gly Ser Thr Pro Ile Ala Asn
       850                 855                 860
   Ala Asp Ala Phe Ile Ala Thr Leu Ser Gly Gln Gly Ala Gly Asn Gln
   865                 870                 875                 880
   Val Thr Val Thr Tyr Lys Glu Glu Asp Asp Val Val Ser Phe Tyr Ile
                   885                 890                 895
   Ile Ser Val Val Asn Gly Gly Ile Gly Asn Pro Thr Thr Asp Pro Ala
               900                 905                 910
   Leu Ala Asn Gly Val Thr Gly Thr Ile Ala Phe Thr Asn Thr Ser Phe
           915                 920                 925
   Asn Ala Ser Ala Asn Gln Thr Ile Thr Val Lys Asp Ala Asp Leu Asn
       930                 935                 940
   Val Asn Ala Thr Val Ala Asp Thr Thr Thr Val Thr Ile Thr Asp Ser
   945                 950                 955                 960
   Ala Lys Thr Thr Phe Asn Ile Thr Leu Thr Glu Thr Gly Val Asn Thr
                   965                 970                 975
   Gly Glu Phe Thr Gly Thr Leu Thr Ser Ala Gln Leu Ala Leu Leu Lys
               980                 985                 990
   Asp Gly Val Ile Thr Ala Thr Tyr Asn Asp Ala Lys Asn Ala Asn Asn
           995                 1000                1005
   Val Ala Ala Thr Ala Thr Ala Thr Ala Thr Leu Asn Thr Thr Val
       1010                1015                1020
   Gly Ala Val Asn Phe Ala Ala Lys Ala Thr Thr Glu Tyr Ser Glu
       1025                1030                1035
   Gly Val Gly Thr Ala Ala Lys Val Glu Gly Ser Thr Val Leu Thr
       1040                1045                1050
   Ser Lys Val Asp Thr Gly Asp Leu Val Leu Val Val Asp Gly Val
       1055                1060                1065
   Gln Phe Thr Thr Val Val Thr Ala Ile Asp Pro Gly Thr Ser Ala
       1070                1075                1080
   Thr Ala Ser Leu Thr Ala Val Val Ala Glu Ile Asn Ala Ala Ala
   1085 1090 1095
   Lys Lys Val Gly Phe Thr Ala Asp Val Ala Thr Val Asp Ala Asp
   1100 1105 1110
   Lys Ile Val Leu Thr Ser Pro Thr Lys Gly Thr Ser Ser Ser Val
   1115 1120 1125
   Glu Val Lys Asp Leu Gly Thr Thr Thr Gly Leu Gly Leu Thr Lys
   1130 1135 1140
   Ala Pro Glu Val Lys Gly Thr Ala Gly Ala Ala Val Ala Thr Gln
   1145 1150 1155
   Trp Lys Phe Thr Val Thr Thr Thr Pro Ala Val Gly Glu Thr Ile
   1160 1165 1170
   Thr Val Lys Val Gly Thr Lys Glu Ala Ser His Lys Val Val Ala
   1175 1180 1185
   Gly Asn Asp Leu Ala Ala Ile Ala Thr Ala Leu Asn Thr Ala Ile
   1190 1195 1200
   Gly Ala Asp Tyr Asn Ile Ala Leu Val Thr Gly Ser Asn Val Ile
   1205 1210 1215
   Thr Val Thr Gln Ala Thr Pro Ala Lys Thr Thr Asp Glu Leu Ser
   1220 1225 1230
   Val Thr Val Thr Lys
   1235
<210> 7
   <211> 3303
   <212> DNA
   <213> Lysinibacillus sphaericus
<220>
   <221> CDS
   <222> (1)..(3303)
<400> 7
   atg gct aac caa cca aag aaa tac aaa aaa ttc gta gcg acg gca gca      48
   Met Ala Asn Gln Pro Lys Lys Tyr Lys Lys Phe Val Ala Thr Ala Ala
   1               5                   10                  15
   acg gca aca tta gta gca tct gct atc gta cca gtg gtt tct gcc gca      96
   Thr Ala Thr Leu Val Ala Ser Ala Ile Val Pro Val Val Ser Ala Ala
               20                  25                  30
   gga ttt tca gat gta gca ggt aac gac cac gaa gta gca atc aac gca     144
   Gly Phe Ser Asp Val Ala Gly Asn Asp His Glu Val Ala Ile Asn Ala
           35                  40                  45
   ctt gct gat gca ggt atc atc aat gga tac gct gac ggc aca ttc aaa     192
   Leu Ala Asp Ala Gly Ile Ile Asn Gly Tyr Ala Asp Gly Thr Phe Lys
       50                  55                  60
   cca aac caa aca att aac cgc ggt caa gtg gtt aaa tta tta ggt cgt      240
   Pro Asn Gln Thr Ile Asn Arg Gly Gln Val Val Lys Leu Leu Gly Arg
   65                  70                  75                  80
   tat tta gaa gca caa ggc caa gaa att cca gct gac tgg aat tca aaa      288
   Tyr Leu Glu Ala Gln Gly Gln Glu Ile Pro Ala Asp Trp Asn Ser Lys
                   85                  90                  95
   caa cgc ttc aac gat cta cca gta aca gct gaa gca gaa tta gta aaa      336
   Gln Arg Phe Asn Asp Leu Pro Val Thr Ala Glu Ala Glu Leu Val Lys
               100                 105                 110
   tat gct gca cta gca aaa gat gca ggc gta ttc aac ggt tca aac ggc      384
   Tyr Ala Ala Leu Ala Lys Asp Ala Gly Val Phe Asn Gly Ser Asn Gly
           115                 120                 125
   aac tta aac gct tca caa aca atg caa cgt caa caa atg gca gta gtt      432
   Asn Leu Asn Ala Ser Gln Thr Met Gln Arg Gln Gln Met Ala Val Val
       130                 135                 140
   tta gta cgt gct att aaa gaa atc gca ggc gta gac tta gta gct gag      480
   Leu Val Arg Ala Ile Lys Glu Ile Ala Gly Val Asp Leu Val Ala Glu
   145                 150                 155                 160
   tac aaa aaa gcg aac ttc gta aca gaa att ggt gac tta gac aaa gct      528
   Tyr Lys Lys Ala Asn Phe Val Thr Glu Ile Gly Asp Leu Asp Lys Ala
                   165                 170                 175
   tac tct gca gaa caa cgc aca gcg atc gtt gct tta gaa tat gcg ggt      576
   Tyr Ser Ala Glu Gln Arg Thr Ala Ile Val Ala Leu Glu Tyr Ala Gly
               180                 185                 190
   att aca aat gtt gct cac ttc aac cca ggt aac agc gta aca cgc gga      624
   Ile Thr Asn Val Ala His Phe Asn Pro Gly Asn Ser Val Thr Arg Gly
           195                 200                 205
   caa ttc gct tca ttc tta tac cgc aca atc gaa aac gtt gta aac aac      672
   Gln Phe Ala Ser Phe Leu Tyr Arg Thr Ile Glu Asn Val Val Asn Asn
       210                 215                 220
   cca gaa gct ggt gtt gcg gca gta aaa gct atc aac aac aca act gtt      720
   Pro Glu Ala Gly Val Ala Ala Val Lys Ala Ile Asn Asn Thr Thr Val
   225                 230                 235                 240
   gaa gta aca ttc gac gaa gaa gta gac aac gta caa gct ctt aac ttc      768
   Glu Val Thr Phe Asp Glu Glu Val Asp Asn Val Gln Ala Leu Asn Phe
                   245                 250                 255
   tta atc tct gat ctt gaa gtg aaa aac gca gct gtt aaa tta aca aac      816
   Leu Ile Ser Asp Leu Glu Val Lys Asn Ala Ala Val Lys Leu Thr Asn
               260                 265                 270
   aaa aaa gtt gtt gta tta aca act gct gct caa aca gct gac aaa gag      864
   Lys Lys Val Val Val Leu Thr Thr Ala Ala Gln Thr Ala Asp Lys Glu
           275                 280                 285
   tac act gta tct ctt ggc gaa gaa aaa atc ggt aca ttc aaa ggt atc      912
   Tyr Thr Val Ser Leu Gly Glu Glu Lys Ile Gly Thr Phe Lys Gly Ile
       290                 295                 300
   gca gct gta gtt cct act aaa gta gat tta gta gaa aaa tca gtt caa      960
   Ala Ala Val Val Pro Thr Lys Val Asp Leu Val Glu Lys Ser Val Gln
   305                 310                 315                 320
   ggt aaa ctt ggc caa caa gta aca ctt aaa gca caa gta act gtt gct     1008
   Gly Lys Leu Gly Gln Gln Val Thr Leu Lys Ala Gln Val Thr Val Ala
                   325                 330                 335
   gaa ggt caa aca aaa gct ggt att cca gta act ttc ttc atc cca ggt      1056
   Glu Gly Gln Thr Lys Ala Gly Ile Pro Val Thr Phe Phe Ile Pro Gly
               340                 345                 350
   tct gct aat ggc gtg aaa act cca gta aca gtt gaa gct gta aca aac      1104
   Ser Ala Asn Gly Val Lys Thr Pro Val Thr Val Glu Ala Val Thr Asn
           355                 360                 365
   gaa gaa ggt atc gca act tac tct tac act cgt tat gct gca aca aac      1152
   Glu Glu Gly Ile Ala Thr Tyr Ser Tyr Thr Arg Tyr Ala Ala Thr Asn
       370                 375                 380
   gac act gta act gta tat gca aac ggt gac cgt tct aaa ttc tca act      1200
   Asp Thr Val Thr Val Tyr Ala Asn Gly Asp Arg Ser Lys Phe Ser Thr
   385                 390                 395                     400
   ggt tat gta ttc tgg gca gtt gac caa act tta gaa atc act gaa gta      1248
   Gly Tyr Val Phe Trp Ala Val Asp Gln Thr Leu Glu Ile Thr Glu Val
                   405                 410                 415
   aca act ggt gct act atc aac aac ggc gct aac aaa act tac aaa gtt      1296
   Thr Thr Gly Ala Thr Ile Asn Asn Gly Ala Asn Lys Thr Tyr Lys Val
               420                 425                 430
   act tac aaa cac cca gaa act ggt aaa cca gta tca ggt aaa gta ctg      1344
   Thr Tyr Lys His Pro Glu Thr Gly Lys Pro Val Ser Gly Lys Val Leu
           435                 440                 445
   aac gtt tca gta aaa gaa aac att gac gta act gtt gat aaa tta caa      1392
   Asn Val Ser Val Lys Glu Asn Ile Asp Val Thr Val Asp Lys Leu Gln
       450                 455                 460
   aac gtt acg gtg aac ggg gta gcg gta gtt caa acg agt aca aac aac      1440
   Asn Val Thr Val Asn Gly Val Ala Val Val Gln Thr Ser Thr Asn Asn
   465                 470                 475                     480
   acg aga gcc gca caa atc aca aca gat tct aga ggt gaa gca aca ttt      1488
   Thr Arg Ala Ala Gln Ile Thr Thr Asp Ser Arg Gly Glu Ala Thr Phe
                   485                 490                 495
   aca gtg tca ggt tcg aac gct gta gtt aca cca gtc gta ttt gaa gct      1536
   Thr Val Ser Gly Ser Asn Ala Val Val Thr Pro Val Val Phe Glu Ala
               500                 505                 510
   act gca acc aat gta gct aac acg tat tct caa aca tac aca tca gat      1584
   Thr Ala Thr Asn Val Ala Asn Thr Tyr Ser Gln Thr Tyr Thr Ser Asp
           515                 520                 525
   gta tta caa gca tct gca gcc aaa gta aca ttt ggt gct att caa gtt      1632
   Val Leu Gln Ala Ser Ala Ala Lys Val Thr Phe Gly Ala Ile Gln Val
       530                 535                 540
   gct tat acg ctt gaa gta acg cgt gat ggt ggt gaa gtg gca gct act      1680
   Ala Tyr Thr Leu Glu Val Thr Arg Asp Gly Gly Glu Val Ala Ala Thr
   545                 550                 555                     560
   ggt gta aca aac ggt cgc aaa tat aac atc gtt gtg aaa gat aag gat      1728
   Gly Val Thr Asn Gly Arg Lys Tyr Asn Ile Val Val Lys Asp Lys Asp
                   565                 570                 575
   ggt aaa gta gca ccg aac gaa atc gtt aac gta gca ttt aac gaa gat      1776
   Gly Lys Val Ala Pro Asn Glu Ile Val Asn Val Ala Phe Asn Glu Asp
               580                 585                 590
   att gat ggc gta att tct aca gtt act tct gca caa ttc gtg aaa gta      1824
   Ile Asp Gly Val Ile Ser Thr Val Thr Ser Ala Gln Phe Val Lys Val
           595                 600                 605
   gaa gac ggt aaa caa gtg ggt tac gaa ggt aaa aaa atc act gtt aga      1872
   Glu Asp Gly Lys Gln Val Gly Tyr Glu Gly Lys Lys Ile Thr Val Arg
       610                 615                 620
   aca aac gct aaa ggt gaa gca agc ttc gtc atc ggc agt gat act gta        1920
   Thr Asn Ala Lys Gly Glu Ala Ser Phe Val Ile Gly Ser Asp Thr Val
   625                 630                 635                 640
   aat tct tac gct aca cca gtc gtt tgg atc gat att aac aac caa agc        1968
   Asn Ser Tyr Ala Thr Pro Val Val Trp Ile Asp Ile Asn Asn Gln Ser
                   645                 650                 655
   ggt aaa gat gca aac tta gac aaa ggt gaa cct tca gca gta gca cca        2016
   Gly Lys Asp Ala Asn Leu Asp Lys Gly Glu Pro Ser Ala Val Ala Pro
               660                 665                 670
   atc tca tac ttc caa gct cca tat tta gac ggt tca aaa tta gta tct        2064
   Ile Ser Tyr Phe Gln Ala Pro Tyr Leu Asp Gly Ser Lys Leu Val Ser
           675                 680                 685
   tat aaa ggt aca act gaa acg gat aaa ttt gca ggt ggc gaa gta gca        2112
   Tyr Lys Gly Thr Thr Glu Thr Asp Lys Phe Ala Gly Gly Glu Val Ala
       690                 695                 700
   aca ttt aaa gta caa ctt aca aac caa agt ggt aaa gtc gta cca aat        2160
   Thr Phe Lys Val Gln Leu Thr Asn Gln Ser Gly Lys Val Val Pro Asn
   705                 710                 715                 720
   tct ggt tac aca acg aag gat gtt acg tac acg gtt tac aat acc ggc        2208
   Ser Gly Tyr Thr Thr Lys Asp Val Thr Tyr Thr Val Tyr Asn Thr Gly
                   725                 730                 735
   gca aac aat ata aaa gtt ggt act gta gaa atc gca cca aac cgc gtg        2256
   Ala Asn Asn Ile Lys Val Gly Thr Val Glu Ile Ala Pro Asn Arg Val
               740                 745                 750
   cat aca gta gtt gcg cca agt ggt gaa att act gtt act act gta gat        2304
   His Thr Val Val Ala Pro Ser Gly Glu Ile Thr Val Thr Thr Val Asp
           755                 760                 765
   ggt aaa act tct tct gta cga gtt tta gct aca ggt gta gcg aaa gag        2352
   Gly Lys Thr Ser Ser Val Arg Val Leu Ala Thr Gly Val Ala Lys Glu
       770                 775                 780
   gtt aac ggg aat aaa gaa ttt gca ttt act tca aaa gaa gca act gca        2400
   Val Asn Gly Asn Lys Glu Phe Ala Phe Thr Ser Lys Glu Ala Thr Ala
   785                 790                 795                 800
   aca ttc act tct act tca gaa atc cct aac cta ttt act ggt gat att        2448
   Thr Phe Thr Ser Thr Ser Glu Ile Pro Asn Leu Phe Thr Gly Asp Ile
                   805                 810                 815
   cat tca ttc gat aca gac aaa gaa act gtc gta ttt act gat ggt tcc        2496
   His Ser Phe Asp Thr Asp Lys Glu Thr Val Val Phe Thr Asp Gly Ser
               820                 825                 830
   gtt cta aaa aat gcg gtg aaa tat gct ggt gaa acg ggc aaa aca tat        2544
   Val Leu Lys Asn Ala Val Lys Tyr Ala Gly Glu Thr Gly Lys Thr Tyr
           835                 840                 845
   gta tat aga aat gta aat ggt gca cca ctt tct aaa gac cag ttt gta        2592
   Val Tyr Arg Asn Val Asn Gly Ala Pro Leu Ser Lys Asp Gln Phe Val
       850                 855                 860
   aat att ctt aaa ggt gcc act tca ggg gta aaa gta aca tat gaa gtt        2640
   Asn Ile Leu Lys Gly Ala Thr Ser Gly Val Lys Val Thr Tyr Glu Val
   865                 870                 875                 880
   aaa gat aat act gtt aca ttc tat gtt gtt tct gtt aac gct ggg gga        2688
   Lys Asp Asn Thr Val Thr Phe Tyr Val Val Ser Val Asn Ala Gly Gly
                   885                 890                 895
   act aag ccg gtt gat att att gga gat aaa gca tct gac gct act gct     2736
   Thr Lys Pro Val Asp Ile Ile Gly Asp Lys Ala Ser Asp Ala Thr Ala
               900                 905                 910
   gtt aca gct gca aaa gct aaa gtt aca gta aca cct aat att gct aca     2784
   Val Thr Ala Ala Lys Ala Lys Val Thr Val Thr Pro Asn Ile Ala Thr
           915                 920                 925
   ctt tca act gat acg gca gaa aat aaa cgt tta gct gta caa tca aat     2832
   Leu Ser Thr Asp Thr Ala Glu Asn Lys Arg Leu Ala Val Gln Ser Asn
       930                 935                 940
   gtt gta act gaa att aat gac tca tca gta act gta agt gta gta gaa     2880
   Val Val Thr Glu Ile Asn Asp Ser Ser Val Thr Val Ser Val Val Glu
   945                 950                 955                 960
   act tct aca gca gga act tat act gtt acg tta act aaa ggc gca agc     2928
   Thr Ser Thr Ala Gly Thr Tyr Thr Val Thr Leu Thr Lys Gly Ala Ser
                   965                 970                 975
   act gct aca gta aat gtt aca cca aaa ttt ggt caa aaa gta act gct     2976
   Thr Ala Thr Val Asn Val Thr Pro Lys Phe Gly Gln Lys Val Thr Ala
               980                 985                 990
   gaa tct att act tct tca ctt gca  gtg aat aca aat gga  aaa gat tta     3024
   Glu Ser Ile Thr Ser Ser Leu Ala  Val Asn Thr Asn Gly  Lys Asp Leu
           995                 1000                 1005
   acc ctt  aat att ggg tct gct  ctt gca ggt ttc aca  atc gcg aat     3069
   Thr Leu  Asn Ile Gly Ser Ala  Leu Ala Gly Phe Thr  Ile Ala Asn
       1010                 1015                 1020
   gta aat  cca gat gct gat aat  gca tta gac att act  act tct ttt     3114
   Val Asn  Pro Asp Ala Asp Asn  Ala Leu Asp Ile Thr  Thr Ser Phe
       1025                 1030                 1035
   aat aat  aat gtg att act ata  gct aaa gca cct tct  gct tct att     3159
   Asn Asn  Asn Val Ile Thr Ile  Ala Lys Ala Pro Ser  Ala Ser Ile
       1040                 1045                 1050
   gaa gct  aca caa tta atc gag  aag act tta aaa gtt  tat ttc gac     3204
   Glu Ala  Thr Gln Leu Ile Glu  Lys Thr Leu Lys Val  Tyr Phe Asp
       1055                 1060                 1065
   gct caa  gct act ggt tat gct  aaa gaa agc ttt gta  gca act att     3249
   Ala Gln  Ala Thr Gly Tyr Ala  Lys Glu Ser Phe Val  Ala Thr Ile
       1070                 1075                 1080
   aca att  gat gat gta acg aac  cgt act ttg gct att  aca gta aag     3294
   Thr Ile  Asp Asp Val Thr Asn  Arg Thr Leu Ala Ile  Thr Val Lys
       1085                 1090                 1095
   cct act  cca                                                  3303
   Pro Thr  Pro
       1100
<210> 8
   <211> 1101
   <212> PRT
   <213> Lysinibacillus sphaericus
<400> 8
   Met Ala Asn Gln Pro Lys Lys Tyr Lys Lys Phe Val Ala Thr Ala Ala
   1               5                   10                  15
   Thr Ala Thr Leu Val Ala Ser Ala Ile Val Pro Val Val Ser Ala Ala
   20 25 30
   Gly Phe Ser Asp Val Ala Gly Asn Asp His Glu Val Ala Ile Asn Ala
   35 40 45
   Leu Ala Asp Ala Gly Ile Ile Asn Gly Tyr Ala Asp Gly Thr Phe Lys
   50 55 60
   Pro Asn Gln Thr Ile Asn Arg Gly Gln Val Val Lys Leu Leu Gly Arg
   65 70 75 80
   Tyr Leu Glu Ala Gln Gly Gln Glu Ile Pro Ala Asp Trp Asn Ser Lys
   85 90 95
   Gln Arg Phe Asn Asp Leu Pro Val Thr Ala Glu Ala Glu Leu Val Lys
   100 105 110
   Tyr Ala Ala Leu Ala Lys Asp Ala Gly Val Phe Asn Gly Ser Asn Gly
   115 120 125
   Asn Leu Asn Ala Ser Gln Thr Met Gln Arg Gln Gln Met Ala Val Val
   130 135 140
   Leu Val Arg Ala Ile Lys Glu Ile Ala Gly Val Asp Leu Val Ala Glu
   145 150 155 160
   Tyr Lys Lys Ala Asn Phe Val Thr Glu Ile Gly Asp Leu Asp Lys Ala
   165 170 175
   Tyr Ser Ala Glu Gln Arg Thr Ala Ile Val Ala Leu Glu Tyr Ala Gly
   180 185 190
   Ile Thr Asn Val Ala His Phe Asn Pro Gly Asn Ser Val Thr Arg Gly
   195 200 205
   Gln Phe Ala Ser Phe Leu Tyr Arg Thr Ile Glu Asn Val Val Asn Asn
   210 215 220
   Pro Glu Ala Gly Val Ala Ala Val Lys Ala Ile Asn Asn Thr Thr Val
   225 230 235 240
   Glu Val Thr Phe Asp Glu Glu Val Asp Asn Val Gln Ala Leu Asn Phe
   245 250 255
   Leu Ile Ser Asp Leu Glu Val Lys Asn Ala Ala Val Lys Leu Thr Asn
   260 265 270
   Lys Lys Val Val Val Leu Thr Thr Ala Ala Gln Thr Ala Asp Lys Glu
   275 280 285
   Tyr Thr Val Ser Leu Gly Glu Glu Lys Ile Gly Thr Phe Lys Gly Ile
   290 295 300
   Ala Ala Val Val Pro Thr Lys Val Asp Leu Val Glu Lys Ser Val Gln
   305 310 315 320
   Gly Lys Leu Gly Gln Gln Val Thr Leu Lys Ala Gln Val Thr Val Ala
   325 330 335
   Glu Gly Gln Thr Lys Ala Gly Ile Pro Val Thr Phe Phe Ile Pro Gly
   340 345 350
   Ser Ala Asn Gly Val Lys Thr Pro Val Thr Val Glu Ala Val Thr Asn
   355 360 365
   Glu Glu Gly Ile Ala Thr Tyr Ser Tyr Thr Arg Tyr Ala Ala Thr Asn
   370 375 380
   Asp Thr Val Thr Val Tyr Ala Asn Gly Asp Arg Ser Lys Phe Ser Thr
   385 390 395 400
   Gly Tyr Val Phe Trp Ala Val Asp Gln Thr Leu Glu Ile Thr Glu Val
   405 410 415
   Thr Thr Gly Ala Thr Ile Asn Asn Gly Ala Asn Lys Thr Tyr Lys Val
   420 425 430
   Thr Tyr Lys His Pro Glu Thr Gly Lys Pro Val Ser Gly Lys Val Leu
   435 440 445
   Asn Val Ser Val Lys Glu Asn Ile Asp Val Thr Val Asp Lys Leu Gln
   450 455 460
   Asn Val Thr Val Asn Gly Val Ala Val Val Gln Thr Ser Thr Asn Asn
   465 470 475 480
   Thr Arg Ala Ala Gln Ile Thr Thr Asp Ser Arg Gly Glu Ala Thr Phe
   485 490 495
   Thr Val Ser Gly Ser Asn Ala Val Val Thr Pro Val Val Phe Glu Ala
   500 505 510
   Thr Ala Thr Asn Val Ala Asn Thr Tyr Ser Gln Thr Tyr Thr Ser Asp
   515 520 525
   Val Leu Gln Ala Ser Ala Ala Lys Val Thr Phe Gly Ala Ile Gln Val
   530 535 540
   Ala Tyr Thr Leu Glu Val Thr Arg Asp Gly Gly Glu Val Ala Ala Thr
   545 550 555 560
   Gly Val Thr Asn Gly Arg Lys Tyr Asn Ile Val Val Lys Asp Lys Asp
   565 570 575
   Gly Lys Val Ala Pro Asn Glu Ile Val Asn Val Ala Phe Asn Glu Asp
   580 585 590
   Ile Asp Gly Val Ile Ser Thr Val Thr Ser Ala Gln Phe Val Lys Val
   595 600 605
   Glu Asp Gly Lys Gln Val Gly Tyr Glu Gly Lys Lys Ile Thr Val Arg
   610 615 620
   Thr Asn Ala Lys Gly Glu Ala Ser Phe Val Ile Gly Ser Asp Thr Val
   625 630 635 640
   Asn Ser Tyr Ala Thr Pro Val Val Trp Ile Asp Ile Asn Asn Gln Ser
   645 650 655
   Gly Lys Asp Ala Asn Leu Asp Lys Gly Glu Pro Ser Ala Val Ala Pro
   660 665 670
   Ile Ser Tyr Phe Gln Ala Pro Tyr Leu Asp Gly Ser Lys Leu Val Ser
   675 680 685
   Tyr Lys Gly Thr Thr Glu Thr Asp Lys Phe Ala Gly Gly Glu Val Ala
   690 695 700
   Thr Phe Lys Val Gln Leu Thr Asn Gln Ser Gly Lys Val Val Pro Asn
   705 710 715 720
   Ser Gly Tyr Thr Thr Lys Asp Val Thr Tyr Thr Val Tyr Asn Thr Gly
   725 730 735
   Ala Asn Asn Ile Lys Val Gly Thr Val Glu Ile Ala Pro Asn Arg Val
   740 745 750
   His Thr Val Val Ala Pro Ser Gly Glu Ile Thr Val Thr Thr Val Asp
   755 760 765
   Gly Lys Thr Ser Ser Val Arg Val Leu Ala Thr Gly Val Ala Lys Glu
   770 775 780
   Val Asn Gly Asn Lys Glu Phe Ala Phe Thr Ser Lys Glu Ala Thr Ala
   785 790 795 800
   Thr Phe Thr Ser Thr Ser Glu Ile Pro Asn Leu Phe Thr Gly Asp Ile
   805 810 815
   His Ser Phe Asp Thr Asp Lys Glu Thr Val Val Phe Thr Asp Gly Ser
   820 825 830
   Val Leu Lys Asn Ala Val Lys Tyr Ala Gly Glu Thr Gly Lys Thr Tyr
   835 840 845
   Val Tyr Arg Asn Val Asn Gly Ala Pro Leu Ser Lys Asp Gln Phe Val
       850                 855                 860
   Asn Ile Leu Lys Gly Ala Thr Ser Gly Val Lys Val Thr Tyr Glu Val
   865                 870                 875                 880
   Lys Asp Asn Thr Val Thr Phe Tyr Val Val Ser Val Asn Ala Gly Gly
                   885                 890                 895
   Thr Lys Pro Val Asp Ile Ile Gly Asp Lys Ala Ser Asp Ala Thr Ala
               900                 905                 910
   Val Thr Ala Ala Lys Ala Lys Val Thr Val Thr Pro Asn Ile Ala Thr
           915                 920                 925
   Leu Ser Thr Asp Thr Ala Glu Asn Lys Arg Leu Ala Val Gln Ser Asn
       930                 935                 940
   Val Val Thr Glu Ile Asn Asp Ser Ser Val Thr Val Ser Val Val Glu
   945                 950                 955                 960
   Thr Ser Thr Ala Gly Thr Tyr Thr Val Thr Leu Thr Lys Gly Ala Ser
                   965                 970                 975
   Thr Ala Thr Val Asn Val Thr Pro Lys Phe Gly Gln Lys Val Thr Ala
               980                 985                 990
   Glu Ser Ile Thr Ser Ser Leu Ala  Val Asn Thr Asn Gly  Lys Asp Leu
           995                 1000                1005
   Thr Leu  Asn Ile Gly Ser Ala  Leu Ala Gly Phe Thr  Ile Ala Asn
       1010                1015                1020
   Val Asn  Pro Asp Ala Asp Asn  Ala Leu Asp Ile Thr  Thr Ser Phe
       1025                1030                1035
   Asn Asn  Asn Val Ile Thr Ile  Ala Lys Ala Pro Ser  Ala Ser Ile
       1040                1045                1050
   Glu Ala  Thr Gln Leu Ile Glu  Lys Thr Leu Lys Val  Tyr Phe Asp
       1055                1060                1065
   Ala Gln  Ala Thr Gly Tyr Ala  Lys Glu Ser Phe Val  Ala Thr Ile
       1070                1075                1080
   Thr Ile  Asp Asp Val Thr Asn  Arg Thr Leu Ala Ile  Thr Val Lys
       1085                1090                1095
   Pro Thr  Pro
       1100
<210> 9
   <211> 386
   <212> PRT
   <213> Lysinibacillus sphaericus
<400> 9
   Tyr Arg Thr Ile Glu Asn Val Val Asn Asn Pro Glu Ala Gly Val Ala
   1               5                   10                  15
   Ala Val Lys Ala Ile Asn Asn Thr Thr Val Glu Val Thr Phe Asp Glu
               20                  25                  30
   Glu Val Asp Asn Val Gln Ala Leu Asn Phe Leu Ile Ser Asp Leu Glu
           35                  40                  45
   Val Lys Asn Ala Ala Val Lys Leu Thr Asn Lys Lys Val Val Val Leu
       50                  55                  60
   Thr Thr Ala Ala Gln Thr Ala Asp Lys Glu Tyr Thr Val Ser Leu Gly
   65                  70                  75                  80
   Glu Glu Lys Ile Gly Thr Phe Lys Gly Ile Ala Ala Val Val Pro Thr
                   85                  90                  95
   Lys Val Asp Leu Val Glu Lys Ser Val Gln Gly Lys Leu Gly Gln Gln
               100                 105                 110
   Val Thr Leu Lys Ala Gln Val Thr Val Ala Glu Gly Gln Thr Lys Ala
           115                 120                 125
   Gly Ile Pro Val Thr Phe Phe Ile Pro Gly Ser Ala Asn Gly Val Lys
       130                 135                 140
   Thr Pro Val Thr Val Glu Ala Val Thr Asn Glu Glu Gly Ile Ala Thr
   145                 150                 155                 160
   Tyr Ser Tyr Thr Arg Tyr Ala Ala Thr Asn Asp Thr Val Thr Val Tyr
                   165                 170                 175
   Ala Asn Gly Asp Arg Ser Lys Phe Ser Thr Gly Tyr Val Phe Trp Ala
               180                 185                 190
   Val Asp Gln Thr Leu Glu Ile Thr Glu Val Thr Thr Gly Ala Thr Ile
           195                 200                 205
   Asn Asn Gly Ala Asn Lys Thr Tyr Lys Val Thr Tyr Lys His Pro Glu
       210                 215                 220
   Thr Gly Lys Pro Val Ser Gly Lys Val Leu Asn Val Ser Val Lys Glu
   225                 230                 235                 240
   Asn Ile Asp Val Thr Val Asp Lys Leu Gln Asn Val Thr Val Asn Gly
   245 250 255
   Val Ala Val Val Gln Thr Ser Thr Asn Asn Thr Arg Ala Ala Gln Ile
   260 265 270
   Thr Thr Asp Ser Arg Gly Glu Ala Thr Phe Thr Val Ser Gly Ser Asn
   275 280 285
   Ala Val Val Thr Pro Val Val Phe Glu Ala Thr Ala Thr Asn Val Ala
   290 295 300
   Asn Thr Tyr Ser Gln Thr Tyr Thr Ser Asp Val Leu Gln Ala Ser Ala
   305 310 315 320
   Ala Lys Val Thr Phe Gly Ala Ile Gln Val Ala Tyr Thr Leu Glu Val
   325 330 335
   Thr Arg Asp Gly Gly Glu Val Ala Ala Thr Gly Val Thr Asn Gly Arg
   340 345 350
   Lys Tyr Asn Ile Val Val Lys Asp Lys Asp Gly Lys Val Ala Pro Asn
   355 360 365
   Glu Ile Val Asn Val Ala Phe Asn Glu Asp Ile Asp Gly Val Ile Ser
   370 375 380
   Thr Val
   385
<210> 10
   <211> 279
   <212> PRT
   <213> Lysinibacillus sphaericus
<400> 10
   Lys Glu Thr Val Val Phe Thr Asp Gly Ser Val Leu Lys Asn Ala Val
   1               5                   10                  15
   Lys Tyr Ala Gly Glu Thr Gly Lys Thr Tyr Val Tyr Arg Asn Val Asn
               20                  25                  30
   Gly Ala Pro Leu Ser Lys Asp Gln Phe Val Asn Ile Leu Lys Gly Ala
           35                  40                  45
   Thr Ser Gly Val Lys Val Thr Tyr Glu Val Lys Asp Asn Thr Val Thr
       50                  55                  60
   Phe Tyr Val Val Ser Val Asn Ala Gly Gly Thr Lys Pro Val Asp Ile
   65                  70                  75                  80
   Ile Gly Asp Lys Ala Ser Asp Ala Thr Ala Val Thr Ala Ala Lys Ala
                   85                  90                  95
   Lys Val Thr Val Thr Pro Asn Ile Ala Thr Leu Ser Thr Asp Thr Ala
               100                 105                 110
   Glu Asn Lys Arg Leu Ala Val Gln Ser Asn Val Val Thr Glu Ile Asn
           115                 120                 125
   Asp Ser Ser Val Thr Val Ser Val Val Glu Thr Ser Thr Ala Gly Thr
       130                 135                 140
   Tyr Thr Val Thr Leu Thr Lys Gly Ala Ser Thr Ala Thr Val Asn Val
   145                 150                 155                 160
   Thr Pro Lys Phe Gly Gln Lys Val Thr Ala Glu Ser Ile Thr Ser Ser
                   165                 170                 175
   Leu Ala Val Asn Thr Asn Gly Lys Asp Leu Thr Leu Asn Ile Gly Ser
               180                 185                 190
   Ala Leu Ala Gly Phe Thr Ile Ala Asn Val Asn Pro Asp Ala Asp Asn
           195                 200                 205
   Ala Leu Asp Ile Thr Thr Ser Phe Asn Asn Asn Val Ile Thr Ile Ala
       210                 215                 220
   Lys Ala Pro Ser Ala Ser Ile Glu Ala Thr Gln Leu Ile Glu Lys Thr
   225                 230                 235                 240
   Leu Lys Val Tyr Phe Asp Ala Gln Ala Thr Gly Tyr Ala Lys Glu Ser
                   245                 250                 255
   Phe Val Ala Thr Ile Thr Ile Asp Asp Val Thr Asn Arg Thr Leu Ala
               260                 265                 270
   Ile Thr Val Lys Pro Thr Pro
           275
<210> 11
   <211> 36
   <212> DNA
   <213> Artificial sequence
<220>
   <223> Oligo
<400> 11
   gacgacgaca agatggcagg attctcagat gtagca 36
<210> 12
   <211> 40
   <212> DNA
   <213> Artificial Sequence
<220>
   <223> Oligo
<400> 12
   gaggagaagc ccggtttatg gagttggctt tactgtaata 40
<210> 13
   <211> 36
   <212> DNA
   <213> Artificial Sequence
<220>
   <223> Oligo
<400> 13
   gacgacgaca agatgatcaa caacacaact gttgaa 36
<210> 14
   <211> 30
   <212> DNA
   <213> Artificial Sequence
<220>
   <223> Oligo
<400> 14
   aaaggatcca tgagtaaagg agaagaactt 30
<210> 15
   <211> 29
   <212> DNA
   <213> Artificial Sequence
<220>
   <223> Oligo
<400> 15
   tttcggccgc tatttgtata gttaatcca 29

## Beschreibung

Die vorliegende Erfindung betrifft röhrenartige biologische Strukturen, die sich aus der äußeren Membran von *E. coli* zusammensetzen und durch heterolog exprimierte rekombinante S-Layer-Proteine stabilisiert werden, Verfahren zu deren Herstellung und deren Verwendung. Die neuartigen Mikroröhren eignen sich für die Anwendung in der Biotechnologie, Pharmazie, in der chemischen Katalyse und in der Nanotechnologie.

S-Layer-Proteine sind bakterielle oder archaeale Hüllproteine, die sich selbst zu regelmäßigen zweidimensionalen Gitterstrukturen (S-Layer) zusammenlagern können (Selbstassemblierung). Diese parakristallinen Gitterstrukturen umfassen regelmäßig angeordnete Poren einheitlicher Größe. Diese spezielle Anordnung führt zu regelmäßigen Abstandsbeziehungen zwischen Molekülgruppen des Monomers wie auch zwischen Metallclustern oder Molekülgruppen, die durch Modifikation an das Monomer gebunden wurden. Damit stehen mit den S-Layer-Proteinen komplexe selbstorganisierende Strukturen zur Verfügung, die mit technischen Mitteln nicht oder nur sehr aufwendig hergestellt werden können (Pollmann u.a. (2007)).

Verschiedene Mikroorganismen verfügen über eine S-Layer-Hülle. Sie produzieren dazu teils sehr unterschiedliche S-Layer-Proteine. Das Molekulargewicht der Monomere kann dabei zwischen 40 und 200 kDa variieren. Die auf ihnen basierenden S-Layer haben in der Regel eine Dicke von 5 bis 20 nm.

Die Fähigkeit zur Selbstassemblierung verleiht den S-Layer-Proteinen ungewöhnliche chemische und strukturelle Eigenschaften, die sie für verschiedenste Anwendungen interessant machen. So lassen sich an S-Layern beispielsweise metallische bzw. Halbleiter-Nanopartikel abscheiden, S-Layer können als Metall-bindende Strukturen oder als Träger für biofunktionale Moleküle verwendet werden, oder sie können zur Umhüllung von Medikamenten eingesetzt werden.

Zur Gewinnung von S-Layer-Proteinen können die Stämme herangezogen werden, die die entsprechenden Proteine natürlicherweise exprimieren (Sleytr, U.B. et al. (2007). Oft sind diese Mikroorganismen jedoch nur schwer kultivierbar und/oder die S-Layer-Isolierung ist sehr aufwendig und damit kostenintensiv. Zur rekombinanten Expression von Proteinen werden daher in der Molekularbiologie häufig im Labor einfach zu kultivierende Mikroorganismen herangezogen, die gentechnisch so modifiziert wurden, dass sie die für das betreffende Protein kodierende DNA-Sequenz enthalten und so das Zielprotein exprimieren. Einer der am häufigsten zu diesem Zweck verwendeten Bakterienstämme ist *Escherichia coli* (*E. coli*) (Pollmann u.a (2007)).

*E. coli* ist ein gram-negatives, peritrich begeißeltes Bakterium, das natürlicherweise im Darm vorkommt und zu den am besten untersuchten Organismen der Welt gehört. Für den labortechnischen Einsatz bei der Expression von rekombinanten Proteinen wurde davon eine Vielzahl an unterschiedlichen Stämmen abgeleitet.

Die Zellen von *E. coli* sind von einer äußeren Membran (*outer membrane*) umgeben. Dies ist eine Besonderheit von gram-negativen Bakterien. Die äußere Membran umgibt die Zellwand der Bakterien als zusätzliche Membran, ist aus Lipopolysacchariden zusammengesetzt und begrenzt das Periplasma. Anders als die Cytoplasmamembran ist sie permeabel für die meisten niedermolekularen Stoffe (Wasser, Ionen, kleine Metabolite) und enthält Porine, die größere Moleküle bis zu 700 Da passieren lassen.

Üblicherweise bilden *E*. *coli*-Zellen Stäbchen von ca. 3 µm x 1 µm aus. In der Wachstumsphase werden häufig kurze Ketten von Einzelzellen beobachtet. Nur in wenigen Publikationen wird von filamentösen *E*. *coli* Zellen berichtet, wie z. B. bei *E*. *coli* str. CS1959, einem Mutanten von *E. coli* K-12 (Parker, C. T. et al., 1992, J. Bacteriol. 174, pp. 2525-2538), oder während Wachstum bei 42°C (Koch, A. L. et al., 1987, J. Bacteriol. 169, pp. 1979-1984) oder unter erhöhter Sauerstoffzufuhr (Preusser, J. H., 1959, Archiv für Mikrobiologie 33, pp. 105-123)(1).

Die der Erfindung zugrundeliegende Aufgabe besteht darin, eine neue Form von biologischen Mikro-Strukturen zur Verfügung zu stellen.

Die Aufgabe wird gelöst durch Mikroröhren, die von *E*. *coli* Zellen gebildet werden, welche in der Lage sind, Filamente zu bilden, und welche S-Layer-Proteine oder Fragmente von S-Layer-Proteinen rekombinant exprimieren. Diese Mikroröhren umfassen die äußere Membran dieser *E*. *coli* Zellen und die von diesen *E*. *coli* Zellen rekombinant exprimierten S-Layer-Proteine bzw. -Proteinfragmente.

Die erfindungsgemäßen Mikroröhren werden aus der äußeren *E. coli* Zellmembran bzw. deren Bestandteilen gebildet. Die Mikroröhren enthalten daher die Proteine und Lipide der äußeren Membran von *E. coli* Zellen. Sie werden durch die Anlagerung von S-Layer-Proteinen oder -Proteinfragmenten stabilisiert, so dass sie auch in der stationären Phase stabil sind und durch Chemikalien wie z. B. 1 % SDS (Natriumdodecylsulfat), Harnstoff oder 3 M NaCl nicht zerstört werden können. Bei den Filamente-bildenden *E*. *coli* Zellen handelt es sich bevorzugt um Zellen des Stammes *E. coli* BI21 (DE3), die natürlicherweise bereits während der exponentiellen Phase lange Filamente ausbilden, die aber später in der stationären Phase wieder in typische *E. coli* Einzelzellen zerfallen.

Die Erfinder haben beobachtet, dass der Stamm *E. coli* BI21 (DE3), der im Stand der Technik für die Expression von rekombinanten Proteinen verwendet wird, in der exponentiellen Phase in Flüssigmedium bei Raumtemperatur lange Filamente von z. T. über 100 µm ausbildet, die aber (wenn die *E*. *coli*-Zellen keine S-Layer-Proteine exprimieren) in der späteren stationären Phase in typische *E. coli* Einzelzellen zerfallen.

Die Erfinder haben überraschend festgestellt, dass, wenn man S-Layer-Proteine oder -Proteinfragmente in diesen filamentbildenden *E*. coli-Zellen exprimiert, die Zellen bisher unbekannte Mikroröhren bilden. Die Mikroröhren bilden sich dabei um die Zellen herum und umschließen eine oder mehrere Zellen. Wenn sich die in den Mikroröhren befindlichen *E*. *coli*-Zellen teilen, vermehrt sich auch das Röhrenmaterial. Die Mikroröhre teilt sich allerdings nicht zusammen mit den *E*. *coli*-Zellen, sondern verlängert sich und hüllt somit sämtliche Tochterzellen ein. Die Mikroröhren werden somit von den *E. coli* Zellen als extrazelluläre Strukturen gebildet, wobei die Zellen ggf. nach Formierung der Mikroröhren entfernt werden.Zellfreie Mikroröhren sind durch Lyse bzw. Herauslocken der Zellen (z. B. durch Chemotaxis) aus den Mikroröhren wie weiter unten beschrieben erhältlich.

Bei den von den *E*. *coli* Zellen rekombinant produzierten S-Layer-Proteinen oder -Proteinfragmenten handelt es sich vorzugsweise um stumme und/oder funktionale S-Layer-Proteine der Bakteriengattung *Lysinibacillus,* um zu diesen verwandte Proteine und/oder Proteinfragmente hiervon.

Bevorzugt stammen die S-Layer-Proteine oder -Proteinfragmente von den Spezies *Lysinibacillus sphaericus,* besonders bevorzugt von den Stämmen *Lysinibacillus sphaericus* JG-A12 (Isolat einer Uranabraumhalde) (Genbank-Akzessionsnummer AJ866975) und/oder *Lysinibacillus sphaericus NCTC* 9602 (Genbank-Akzessionsnummer AJ866974).

Die von den *E. coli* Zellen rekombinant produzierten S-Layer-Proteine haben bevorzugt eine Aminosäuresequenz, die ausgewählt ist aus den Aminosäuresequenzen der S-Layer-Proteine SlfA **(SEQ ID-No. 2),** SllA **(SEQ ID-No. 4),** SlfB **(SEQ ID-No. 6)** oder SllB **(SEQ ID-No. 8)** oder einer Teilsequenz davon mit einer Länge von mindestens 100 Aminosäuren, vorzugsweise mindestens 150 Aminosäuren, bevorzugt mindestens 200 Aminosäuren, besonders bevorzugt mindestens 250 Aminosäuren, oder eine zu mindestens einer dieser Sequenzen homologe Sequenz mit mindestens 70 % Sequenzidentität, bevorzugt 80 % Sequenzidentität, besonders bevorzugt 90 % Sequenzidentität.

Die erfindungsgemäßen Mikroröhren sind erhältlich durch die Expression von einer für ein S-Layer-Protein, bevorzugt ein S-Layer-Protein von *Lysinibacillus sphaericus* JG-A12, kodierenden Nukleinsäuresequenz oder einer Teilsequenz davon in einer Filamente-bildenden *E*. *coli* Wirtszelle.

Ganz besonders bevorzugt handelt es sich bei den von den *E. coli* Zellen rekombinant exprimierten Nukleinsäuresequenzen um die für S-Layer-Proteine kodierenden Gene *slfA* **(SEQ ID-No. 1;** Genbank-Akzessionsnr. AJ849547), *sllA* **(SEQ ID-No. 3;** Genbank-Akzessionsnr. AJ849548), *slfB* **(SEQ ID-No. 5;** Genbank-Akzessionsnr. AJ849549) und/oder *sllB* **(SEQ ID-No. 7;** Genbank-Akzessionsnr. AJ849550) von *Lysinibacillus sphaericus* JG-A12.

Für die Bildung von Mikroröhren ist insbesondere der Sequenzbereich verantwortlich, der dem Bereich zwischen 650 bp und dem Stoppcodon, besonders den Sequenzbereichen zwischen den Positionen 650 bp und 1800 bp und zwischen den Positionen 2473 bp und dem Stoppcodon einer der Sequenzen ausgewählt von SEQ ID-No. 1, 3, 5 oder 7 befindet.

Die entsprechenden Bereiche der S-Layer-Proteine für die Mikroröhrenbildung liegen also in dem Bereich, der dem Abschnitt von 217 Aminosäuren bis zum C-terminalen Ende, besonders den Abschnitten von 217 bis 600 Aminosäuren und von 824 Aminosäuren bis zum C-terminalen Ende einer der Sequenzen gemäß den SEQ ID-No. 2, 4, 6 oder 8 entspricht.

Werden also Mikroröhren mit Teilsequenzen der S-Layer-Proteine erzeugt, umfassen diese mindestens einen der Bereiche, die von 217 bis 600 Aminosäuren bzw. von 824 Aminosäuren bis zum C-terminalen Ende einer der Sequenzen gemäß den SEQ ID-No. 2, 4, 6 oder 8 reichen, oder eine zu mindestens einer dieser Sequenzen homologe Sequenz mit mindestens 70 % Sequenzidentität, bevorzugt 80 % Sequenzidentität, besonders bevorzugt 90 % Sequenzidentität.

Besonders bevorzugt umfassen die Teilsequenzen die entsprechenden Abschnitte der Aminosäuresequenzen gemäß den SEQ ID-No. 2 und/oder 8 oder eine zu mindestens einer dieser Sequenzen homologe Sequenz mit mindestens 50 % Sequenzidentität, vorzugsweise mindestens 70 % Sequenzidentität, bevorzugt 80 % Sequenzidentität, besonders bevorzugt 90 % Sequenzidentität.

Ganz besonders bevorzugt umfassen sie die folgenden Aminosäuresequenzen SEQ ID-No. 9 und 10, die den Abschnitten von 217 bis 600 Aminosäuren (SEQ ID-No. 9) bzw. von 824 bis 1101 Aminosäuren (SEQ ID-No. 10) des S-Layer-Proteins SllB aus *Lysinibacillus sphaericus* JG-A12 entsprechen, oder eine zu mindestens einer dieser Sequenzen homologe Sequenz mit mindestens 70 % Sequenzidentität, bevorzugt 80 % Sequenzidentität, besonders bevorzugt 90 % Sequenzidentität.

Werden in die Zellen des Filamente-bildenden *E. coli* Stammes Gene kloniert, die für S-Layer-Gene z. B. von *Lysinibacillus sphaericus* (z.B. *sll*A, *sll*B, *slf*A, *slf*B) kodieren und werden diese S-Layer-Proteine exprimiert, werden die filamentösen Strukturen der exponentiellen Phase dadurch stabilisiert.

Bestandteil der Erfindung ist daher auch die Verwendung einer Nukleinsäuresequenz ausgewählt aus den SEQ-ID No. 1, 3, 5 oder 7, einer Teilsequenz davon oder einer zu mindestens einer dieser Sequenzen homologen Nukleinsäuresequenz mit mindestens 70 % Sequenzidentität, bevorzugt 80 % Sequenzidentität, besonders bevorzugt 90 % Sequenzidentität, zur Herstellung der erfindungsgemäßen Mikroröhren, sowie die entsprechende Verwendung einer *E*. *coli*-Zelle, die eine dieser Sequenzen umfasst.

Erreichen die *E*. *coli*-Zellen die stationäre Phase, so sind lange röhrenartige Strukturen zu beobachten, in denen Einzelzellen von *E. coli* enthalten sind. Diese Strukturen ähneln in ihrer Zusammensetzung der äußeren Membran von *E. coli.* Die rekombinant exprimierten S-Layer-Proteine sind mit dieser röhrenförmigen Membranstruktur assoziiert und stabilisieren diese. Die erfindungsgemäßen Mikroröhren sind chemisch äußerst stabil und lassen sich durch Chemikalien wie z.B. 1 % SDS, Harnstoff oder 3 M NaCl kaum oder gar nicht zerstören.

Durch die hohe Stabilität der Mikroröhren erhalten die erfindungsgemäßen Mikroröhren produzierenden Bakterienzellen vorteilhaft ebenfalls eine erhöhte mechanische Stabilität. Die Mikroröhren-bildenden Zellen sind mit herkömmlichen Verfahren wie Ultraschall oder Hochdruckhomogenisation nicht oder nur sehr schwer zu zerstören. Dies kann vorteilhaft genutzt werden, wenn eine Lyse der Zellen durch mechanische Behandlung vermieden werden soll.

Die erfindungsgemäßen Mikroröhren erreichen eine Länge von teilweise über 100 µm. Vorzugsweise sind sie zwischen 5 µm und 200 µm, bevorzugt zwischen 10 µm und 100 µm, besonders bevorzugt zwischen 20 µm und 80 µm lang. Ihr Querschnitt ist durch den Zelldurchmesser der von-ihnen umgebenen Bakterien beeinflusst. Vorzugsweise liegt er zwischen 0,4 µm bis 2 µm, bevorzugt zwischen 0,5 µm und 1 µm.

Die erfindungsgemäßen Mikroröhren können die Bakterien, von denen sie gebildet wurden enthalten. Vorzugsweise sind die Bakterien aber durch Behandlung mit verschiedenen Chemikalien entfernt. Ob die Bakterien stören oder nicht ist jedoch abhängig vom Verwendungszweck.

Da die Mikroröhren sehr stabil gegen verschiedene Chemikalien sind, ist die Entfernung der in ihnen enthaltenen Bakterienzellen nicht ohne weiteres möglich.

Bestandteil der Erfindung ist daher auch ein Verfahren zur Isolierung der erfindungsgemäßen Mikroröhren und zur Entfernung der *E. coli* Zellen aus den Mikroröhren. Vorzugsweise werden dazu die Mikroröhren mit den darin enthaltenen Zellen in einer wässrigen Lösung eines Mono- und/oder Disaccharids oder einer wässrigen Ethanollösung inkubiert. Bevorzugt wird das Mono- und/oder Disaccharid ausgewählt aus Glucose, Fructose, Galactose, Saccharose, Maltose, und Lactose. Besonders bevorzugt ist Saccharose.

Enthält die wässrige Lösung Ethanol, liegt der Anteil an Ethanol bevorzugt bei 5 % bis 20 %, besonders bevorzugt bei 7,5 % bis 17,5 %, ganz besonders bevorzugt bei 9 % bis 15 %.

Enthält die wässrige Lösung ein oder mehrere Mono- und/oder Disaccharide, ist der Gesamtanteil der Mono- und/oder Disaccharide bevorzugt 20 Gew.-% bis 60 Gew.-%, besonders bevorzugt 30 Gew.-% bis 50 Gew.-%, ganz besonders bevorzugt 35 Gew.-% bis 45 Gew.-%. Durch diese Behandlung verlässt ein Großteil der *E. coli* Zellen die Mikroröhren. Vorzugsweise werden sowohl die Mikroröhren als auch die Zellen anschließend mit einer hochkonzentrierten Harnstofflösung behandelt. Durch diesen zweiten Reinigungsschritt, werden die noch in den Röhren verbliebenen Zellen zum Verlassen der Röhren gezwungen. Vorzugsweise beträgt die Konzentration der Harnstofflösung zwischen 4 mol/l und 8 mol/l, bevorzugt zwischen 5 mol/l und 7 mol/l.

Vorteilhaft wachsen die die S-Layer-Proteine rekombinant exprimierenden, Mikroröhren-bildenden *E*. *coli*-Zellen nach Induktion der S-Layer-Produktion unter Standard-Wachstumsbedingungen, wie sie in Standardwerken wie z. B. Sambrook et al. (1989) beschrieben werden, zu sehr hohen Zelldichten von bis zu A₆₀₀ = 5,0 heran. Damit eignen sie sich zur effizienten Produktion von rekombinanten Proteinen. Als Wachstumsbedingungen werden dabei bevorzugt zwischen 50 bis 100 ml LB(Luria-Bertani)-Medium in geeigneten Erlenmeyer-Schüttelkolben (beispielsweise einer Größe von 100 ml bis 300 ml), und Schüttelfrequenzen zwischen 200 bis 300 Umdrehungen/min, vorzugsweise von 220 bis 250 Umdrehungen/min gewählt.

Die Produktion der Mikroröhren durch die *E*. *coli*-Zellen erfolgt vorzugsweise bei Temperaturen unter 37 °C, bevorzugt bei weniger als 25 °C, besonders bevorzugt zwischen 10 und 14 °C.

Bestandteil der Erfindung sind auch Mikroröhren, die metallisiert sind. Die hergestellten Röhrenstrukturen zeigen sehr gute Bindungseigenschaften für unterschiedliche Metalle, wie z.B. Au(III) oder Pd(II). So beträgt die Bindungskapazität für Pd(II) 8 mg/g Biomasse, für Au(III) 14 mg/g Biomasse und für Pb(II) 8,5 mg/g Biomasse. Eine erfindungsgemäße Anwendung ist daher die Herstellung von dünnen metallischen Mikroröhren, beispielsweise Gold- oder Palladium-Mikroröhren, für die Elektronik, indem die Mikroröhren als Matrix für eine Metallisierung verwendet werden.

Die erfindungsgemäßen metallisierten Mikroröhren sind durch Inkubation in einer Metallsalzlösung (z. B. Au(III)) und anschließende Zugabe eines Reduktionsmittels (z.B. DMAB) erhältlich.

Da eine preiswerte Biomassegewinnung durch das Erreichen hoher Zelldichten sehr gut möglich ist, sind die Mikroröhren außerdem vorteilhaft für eine preiswerte Entfernung von Metallen aus (Ab-)Wässern geeignet. Bestandteil der Erfindung ist daher auch die Verwendung der Mikroröhrenstrukturen als metallbindende Filtermaterialien.

Die erfindungsgemäßen Mikroröhren eignen sich außerdem für eine Funktionalisierung, indem die rekombinant exprimierten S-Layer-Proteine mit Peptiden oder Proteinen fusioniert und diese Peptide und Proteine durch die Fusion mit den S-Layer-Proteinen an die Mikroröhren gebunden werden. Die Mikroröhren können auf diese Weise beispielsweise mit Enzymen funktionalisiert und als Katalysatoren verwendet werden. Denkbar ist auch die Verwendung der funktionalisierten Röhren zur Herstellung von Antikörpern.

Die neuartigen Mikroröhren eignen sich aufgrund ihrer faserartigen Struktur auch zur Verwendung in Verbundmaterialien und/oder Textilien.

Prinzipiell eignen sich die Mikroröhren zur Verwendung als Carrier (Träger) für pharmazeutische Wirkstoffe. Eine andere Anwendungsmöglichkeit betrifft die Verwendung der Mikroröhrenstrukturen als Container, Umhüllung bzw. als Carrier (Träger) für heterolog koexprimierte Proteine wie z.B. Enzyme. Dabei werden zusätzlich zu den S-Layer-Proteinen, die für die Stabilisierung der Mikroröhrenstruktur sorgen, andere rekombinante Proteine exprimiert, die in den Mikroröhren auch nach Zerstörung der Zellen verbleiben und so immobilisiert sind.

Die Erfindung wird durch die folgenden Figuren und Ausführungsbeispiele näher erläutert, ohne auf diese beschränkt zu sein.

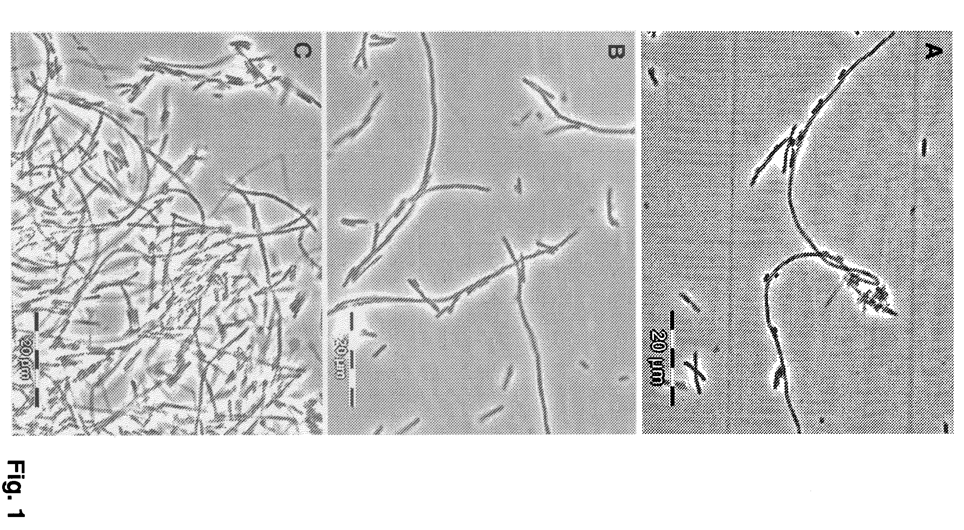

**Fig. 1** **A bis C** enthält verschiedene lichtmikroskopische Aufnahmen von S-Layer-Protein exprimierenden *E. coli* BI21(DE3), die mit einem Olympus BX61-Mikroskop (Olympus Optical, Europe; GmbH, Hamburg) im Phasenkontrastverfahren aufgenommen wurden, und zeigt die Mikroröhrenbildung nach der Induktion der S-Layer-Protein-Expression durch IPTG.

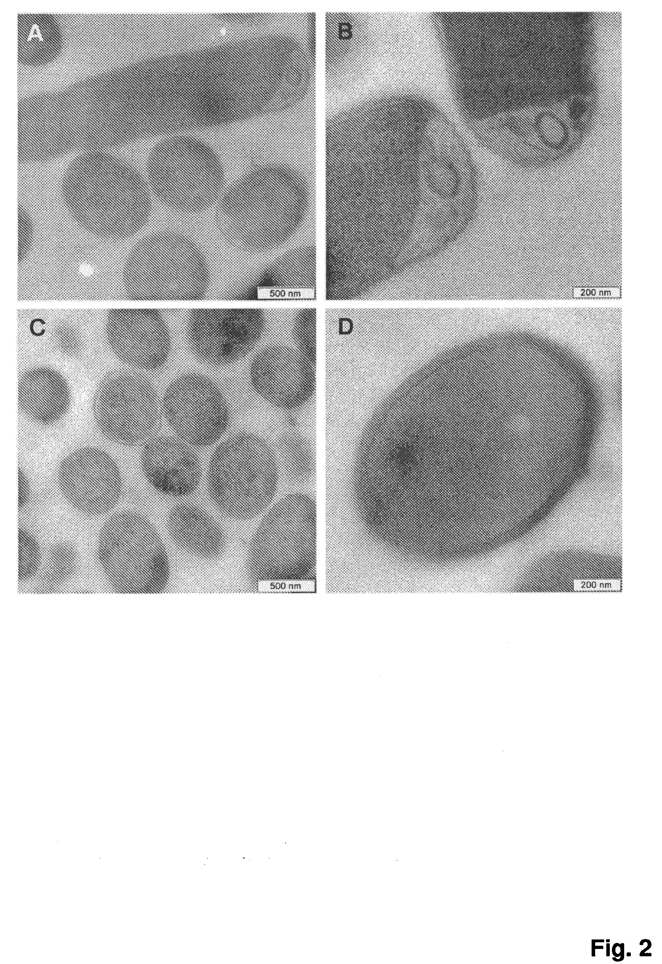

**Fig. 2** **A** und **B** zeigen transmissionselektronenmikroskopische (TEM-)Aufnahmen von *SllB-*exprimierenden *E. coli* BI21(DE3)-Zellen. Als Vergleich dienen **Fig. 2** **C** und **D,** die Aufnahmen von *E. coli* BI21(DE3)-Zellen zeigen, die kein für *SllB* kodierendes Plasmid enthalten. Die Aufnahmen wurden mit einem EM 912 OMEGA Transmissionselektronenmikroskop (LEO Elektronenmikroskopie GmbH, Oberkochen, Deutschland) erstellt.

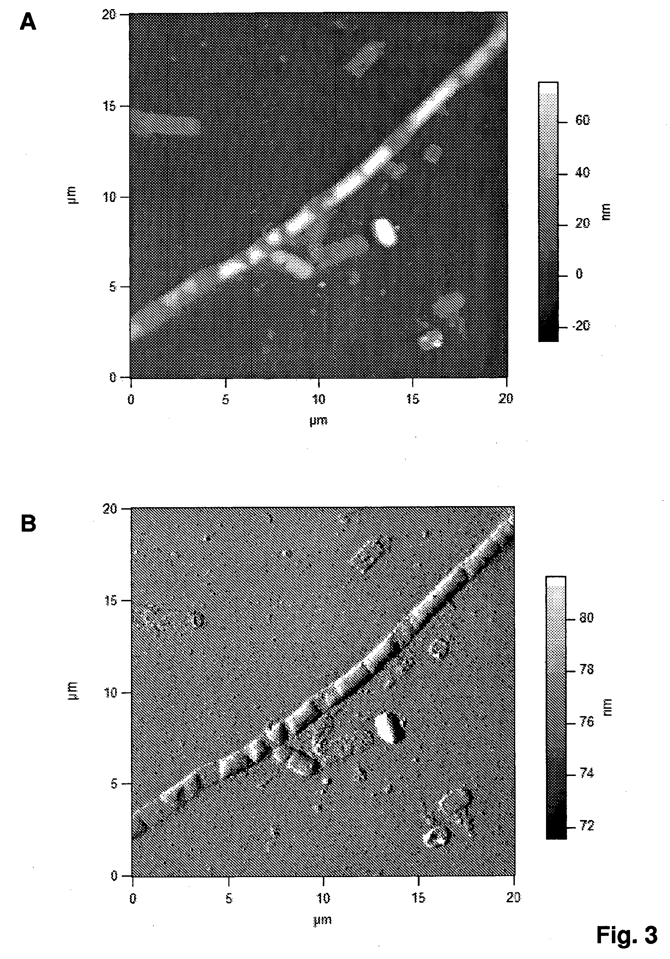

**Fig. 3** **A, B** zeigt mit einem Rasterkraftmikroskop aufgenommene Bilder einer mit *E. coli* BI21(DE3)-Zellen gefüllten Mikroröhre. Die AFM-Bilder wurden mit dem Rasterkraftmikroskop MFP3D (Bio Asylum Research, Santa Barbara, Canada) aufgenommen.

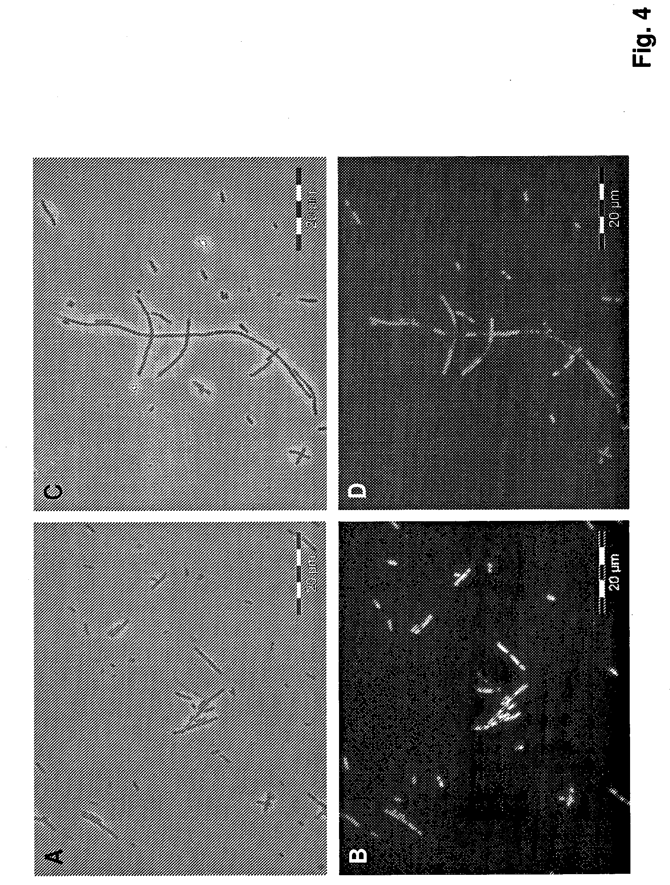

**Fig. 4** zeigt lichtmikroskopisch (Phasenkontrast) **(A, C)** und fluoreszenzmikroskopisch aufgenommene Bilder einer *E. coli* BI21(DE3)-Zellkultur, die das SllB-GFP-Fusionsprotein exprimiert **(B, D).** Die Bilder wurden mit dem Olympus BX61-Mikroskop (Olympus Optical, Europe; GmbH, Hamburg) unter Verwendung des GFP-Filters U-MNIBA2 (Olympus Optical, Europe; GmbH, Hamburg) mit einer Belichtungszeit von 100 ms aufgenommen.

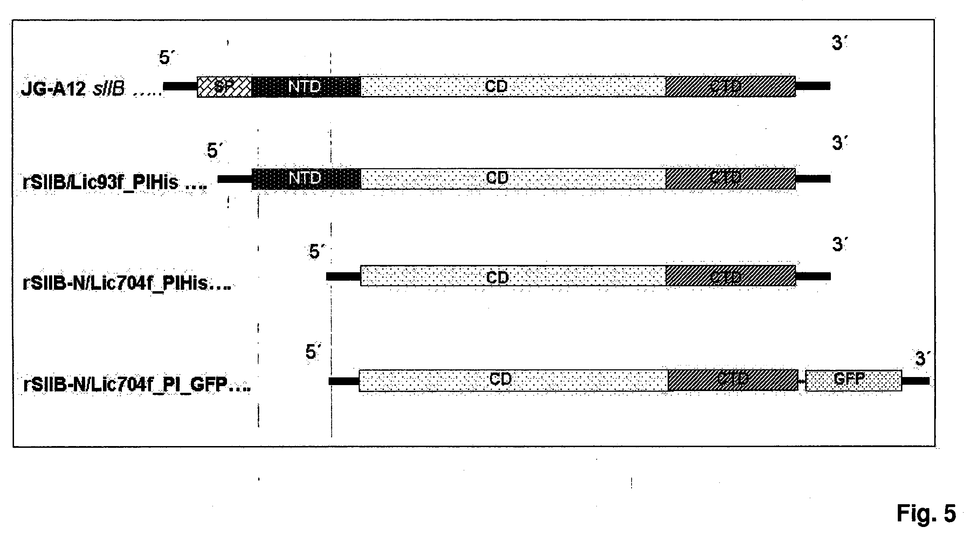

**Fig. 5** Schematischer Aufbau der Konstrukte für die Expression von verschiedenen rekombinanten (Fusions)proteinen. Ganz oben ist das gesamte *sllB*-*Gen* einschließlich der für das Signalpeptid kodierenden Sequenz dargestellt und zeigt den grundsätzlichen Aufbau des Gens, das als Templat für die PCR(Polymerase Chain Reaction)-Amplifikation verwendet wurde. SP = Signalpeptid; NTD = N-terminale Domäne; CD = Zentrale Domäne; CTD = C-terminale Domäne.

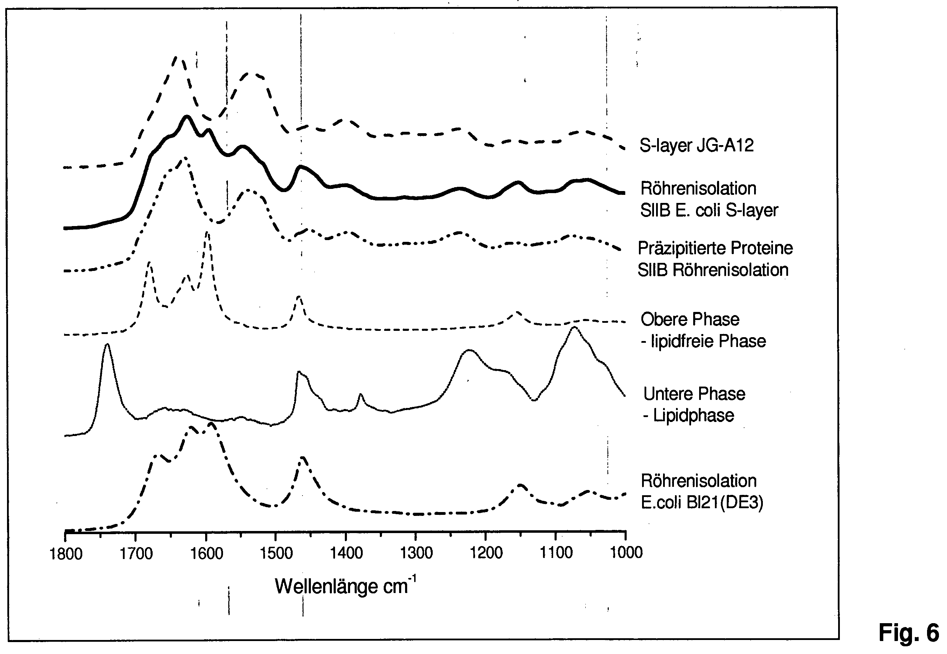

**Fig. 6** zeigt IR-Spektren verschiedener Röhren- oder Membranfraktionen während der Aufreinigung der Mikroröhren bestehend aus den *E*. coli-Röhrenkomponenten und dem S-Layer Protein SllB, sowie im Vergleich dazu das S-Layer-Spektrum von SlfB, welches ein dem SIIB vergleichbares Spektrum zeigt. Von oben nach unten sind dies: reines JG-A12 S-Layer-Protein (Kontrolle), Röhrenisolat von SllB-exprimierenden *E. coli* BI21 (DE3)-Zellen, bei Lipidextration aus dem Röhrenisolat ausgefällte Proteinfraktion, obere, lipidfreie Phase, untere Lipidphase, und Röhrenisolat von *E. coli* BI21 (DE3)-Zellen, die kein S-Layer-Protein exprimieren (Kontrolle).

### Ausführungsbeispiele

### Ausführungsbeispiel 1: Herstellung von Zellen, die die erfindungsgemäßen Mikroröhrenstrukturen exprimieren

*Lysinibacillus sphaericus* JG-A12 ist ein natürliches Isolat von der Uranabraumhalde Haberland nähe Johanngeorgenstadt, Deutschland (Selenska-Pobell et al. 1999). Die Bakterien werden üblicherweise in NB-(nutrient broth)-Medium kultiviert. *Escherichia coli* NovaBlue GigaSingles (Novagen) und *Escherichia coli* BI21 (DE3) (Novagen) sind von *E*. *coli* K12 abgeleitete Stämme und werden üblicherweise in LB(Luria-Bertani)-Medium kultiviert.

Als Templat für die PCR-Amplifikation wird genomische DNA von *Lysinibacillus sphaericus* JG-A12 isoliert und aufgereinigt.

Für die Erzeugung eines N-terminal His-getaggten (d. h. mit einem His-Tag versehenen) rekombinanten Proteins (rSllB/Lic93f_PIHis, **Fig. 5****,** zweite von oben) wird das S-layer-Protein kodierende Gen *sllB* von der chromosomalen DNA von *L. sphaericus* JG-A12 mit dem Primerpaar Lic93f **(SEQ ID-No. 11;** 5'-GAC GAC GAC AAG ATG/GCA GGA TTC TCA GAT GTA GCA-3') und Lic_PIHis **(SEQ ID-No. 12;** 5'-GAG GAG AAG CCC GGT/TTA TGG AGT TGG CTT TAC TGT AAT A-3') amplifiziert.

Für die Erzeugung eines N-terminal verkürzten und His-getaggten rekombinanten Proteins (rSllB-N/Lic704f_PIHis, **Fig. 5****,** dritte von oben) wird das S-Layer-Protein kodierende Gen *sllB* von der chromosomalen DNA von *L. sphaericus* JG-A12 mit dem Primerpaar Lic704f **(SEQ ID-No. 13;** 5'-GAC GAC GAC AAG ATG/ATC AAC AAC ACA ACT GTT GAA-3') und Lic_PIHis amplifiziert.

Positive PCR-Produkte, die für das stille S-layer-Protein bzw. das N-terminal verkürzte Fragment kodieren, werden in den Vektor pET-30 Ek/LIC (Novagen) kloniert und das erhaltene Konstrukt in die nicht exprimierende Wirtszelle *Escherichia coli* NovaBlue (Novagen) transformiert. Positive Klone werden isoliert und in *E. coli* BI21 (DE3) als exprimierende Wirtszelle transformiert.

Für die Erzeugung eines N-terminal His-getaggten rekombinanten SllB-GFP-Fusionsproteins (rSllB-N/Lic704f_PI_GFP, **Fig. 5****,** ganz unten) wird das GFP-Gen mit dem Primerpaar Lic_GFP_BamHI_F **(SEQ ID-No. 14;** 5'-AAAGGATCCATGAGTAAAGGAGAAGAACTT-3') und Lic_GFP_Eagl_R **(SEQ ID-No. 15;** 5'-TTTCGGCCGCTATTTGTATAGTTAATCCA-3') von dem pGFP-Vektor (Clontech) amplifiziert. Anschließend werden die PCR-Produkte des Primerpaars Lic_GFP_BamHI_F und Lic_GFP_Eagl_R C-terminal in das aufgereinigte und restriktionsverdaute Plasmid rSllB-N/Lic704f_PI ligiert und in *Escherichia coli* NovaBlue (Novagen) als nichtexprimierende Wirtszelle transformiert. Positive Klone werden identifiziert, isoliert und in *E. coli* BL21 (DE3) als exprimierende Wirtszelle transformiert.

### Ausführungsbeispiel 2. Analyse der Expressionsprodukte

Für die verschiedenen SllB-Fusionsproteine, nämlich a) SIIB ohne Signalpeptid und mit N-terminalem His-tag (rSllB/Lic93f_PIHis) **(****Fig. 5****,** zweite von oben), b) N-terminal verkürztes SIIB mit N-terminalem His-tag (rSllB-N/Lic704f_PIHis) (**Fig**. **5****,** dritte von oben) und c) N-terminal verkürztes SIIB mit C-terminaler GFP-Fusion und N-terminalem His-tag (rSllB-N/Lic704f_PI_GFP) **(****Fig. 5****,** ganz unten), kodierende Plasmide werden wie in Ausführungsbeispiel 1 beschrieben erzeugt, die rekombinanten Proteine exprimiert und die Expression anschließend durch SDS-PAGE überprüft.

Für die Expression werden 100 ml of Luria-Bertani(LB)-Medium mit Kanamycin (25 µg/ml) versetzt und mit 5 ml einer in LB gezogenen Vorkultur des mit dem entsprechenden Plasmid transformierten *E. coli* BI21 (DE3)-Stammes beimpft. Die Kulturen werden bei Raumtemperatur inkubiert. Nach zwei Stunden wird die rekombinante Genexpression durch die Zugabe von 0.1 mmol/l Isopropylthiogalactosid (IPTG) induziert.

Die Zellen werden nach der Inkubation über Nacht in der stationären Phase geerntet, indem sie 5 min bei 6000 x g abzentrifugiert und zweimal in Resuspensionspuffer (100 mmol/l NaH₂PO₄xH₂O; 10 mmol/l Tris-Base; 0.5 m NaCl, pH=8.0) gewaschen werden. Nach der Resuspension in 5 ml Puffer werden die Zellen durch jeweils 5 bis 6 halbminütige Ultraschallbehandlung bei 60 % Amplitude aufgeschlossen (Sonifier W250-D, Branson). Das Lysat wird 30 min bei 30,000 x *g* abzentrifugiert und der Überstand abgenommen und bei 4 °C für die weitere Untersuchung aufbewahrt. Die abzentrifugierten Zelltrümmer werden in Resuspensionspuffer resuspendiert.

Das Proteinprofil des Überstandes, der die löslichen Proteine enthält, und der abzentrifugierten Zelltrümmer, wird durch SDS-PAGE (sodium dodecyl sulfatepolyacrylamide gel electrophoresis) überprüft. Die SDS-PAGE wird ausgeführt wie von Laemmli (1970) beschrieben. Im Vergleich zu Zellextrakten von nicht-transformierten *E. coli* BI21 (DE3) können in allen Proben zusätzliche hochmolekulare Proteinbanden beobachtet werden, die in etwa den berechneten Molekulargewichten der Fusionsproteine rSllB/Lic93f_PIHis (-118 kDa), rSllB-N/Lic704f_PIHis (-96 kDa) und rSllB-N/Lic704f_PI_GFP (-124 kDa) **(****Fig. 5****)** entsprechen.

Die Expression von SllB wird durch Immunblot unter Verwendung von polyklonalem primärem Antikörper, der gegen das S-layer-Protein von *L. sphaericus* JG-A12 (Pineda Antikörper Service, Berlin) erzeugt worden war, und einem Immun-Blot Assay Kit (BioRad, Hercules, USA) mit geeignetem sekundärem Antikörper analysiert. Auf den Immunblots ist nach der Inkubation mit sekundärem Anti-Kaninchen-Antikörper konjugiert mit Alkaliner Phosphatase eine starke Kreuzreaktion zwischen den in der SDS-Page identifizierten hochmolekularen Proteinbanden und dem polyklonalen-Kaninchen-Antiserum, das gegen SlfB von *Lysinibacillus sphaericus* JG-A12 erzeugt worden war, zu beobachten.

Die rekombinanten Proteine werden mit kommerziell erhältlichem Proteinfarbstoff (SYPRO-Ruby Protein Gel stain; BioRad, Hercules, USA) und einem kommerziell erhältlichen Kit zur Bestimmung des Proteingehalts (BioRad Protein Assay; BioRad, Hercules, USA) quantifiziert. Die Konzentration der Fusionsproteine wird mittels des kommerziell erhältlichen Proteinfarbstoffs sowie über die Bradford-Methode bestimmt. Die 100 ml-Kultur von mit rSllB/Lic93f_PIHis transformierten *E*. *coli* BI21 (DE3) enthält ∼14.5 mg Fusionsprotein (-25 % des Gesamtproteingehalts), die 100 ml-Kultur von mit rSllB-N/Lic704f_PIHis transformierten *E. coli* BI21 (DE3) enthält -18 mg Fusionsprotein (-36 % des Gesamtproteingehalts) und die 100 ml-Kultur von mit rSllB-N/Lic704f_PI_GFP transformierten *E. coli* BI21 (DE3) enthält -12 mg Fusionsprotein (-18 % des Gesamtproteingehalts).

### Ausführungsbeispiel 3: Vorschrift zur Isolierung von Mikroröhren

Zunächst wird das stumme S-Layer Gen *sllB* des Stammes *Lysinibacillus sphaericus* JG-A12 (Genbank-Akzessionsnummer AJ849550) unter Verwendung von genspezifischen Primern durch PCR wie in Ausführungsbeispiel 1 beschrieben vervielfältigt. Das erhaltene PCR-Produkt mit einer Länge von 3210 bp wird in den Vector pET-30 Ek/Lic (Novagene) ligiert. Der Vector mit dem *sllB*-Insert wird zunächst in *E. coli* NovaBlue (Novagene) transformiert. Von positiven Klonen werden Plasmide isoliert, die in den Expressionsstamm *E. coli* BI21(DE3) transformiert werden.

Für die Expression von *sllB* wird zunächst eine Vorkultur in LB-Medium angezogen. Diese wird in frisches LB-Medium überimpft und bis zu einer O.D. von ca. 0,1 inkubiert. Das Wachstum erfolgt bei Raumtemperatur (< 25°C). Nach ca. 2 h Wachstum erfolgt eine Zugabe von 0,1 mmol/l IPTG und damit eine Induktion der Expression der rekombinanten SIIB Proteine. In der exponentiellen Phase erfolgt eine Bildung von langen Filamenten von E. *coli*-Zellen. In der späten exponentiellen Phase und stationären Phase sind lange Mikroröhren einer Länge von z. T. > 100 µm zu beobachten, in denen Einzelzellen von *E. coli* zu erkennen sind.

Eine stabile Mikroröhrenbildung erfolgt nur in Flüssigkultur bei genügender Sauerstoffzufuhr und Temperaturen <25 °C. Auf Agar-Platten bilden die Bakterien normale Einzelzellen aus.

Die Isolierung der Mikroröhren erfolgt folgendermaßen: Nach der Ernte der Zellen durch Zentrifugation (10,000 x g, 10 min) werden die Zellen in 1x PBS Puffer resuspendiert und zweimal mit destilliertem H₂O oder in 1xPBS-Puffer gewaschen. Die Überstände werden verworfen. Anschließend werden die Zellen mit 40 % Saccharose versetzt und für 1 h bei RT geschüttelt. Dabei verlassen die Zellen die Mikroröhren. Die Zellen werden 15 min bei 3000 x g und 4 °C abzentrifugiert und der Überstand mit den Mikroröhren wird abgenommen. Der Überstand wird anschließend 30 min bei 12000 x *g* zentrifugiert. Das Pellet mit den Mikroröhren wird mit 6 mol/l Harnstoff versetzt und 1 h bei Raumtemperatur inkubiert. Die Zellen werden ebenfalls in 6 mol/l Harnstoff resuspendiert und für 1 h geschüttelt. Beide Fraktionen werden erneut bei 3000 x *g* zentrifugiert. Die Mikroröhren befinden sich im Überstand, aus dem sie bei 12000 x *g* abzentrifugiert werden. Das Pellet mit den Mikroröhren wird mehrfach in dest. H₂O gewaschen und bei 12000 x g abzentrifugiert, und in destilliertem Wasser aufgelöst und aufbewahrt.

Wie durch die Ansequenzierung des N-Terminus festgestellt wurde, enthalten die Mikroröhren verschiedene Proteine der äußeren Bakterienmembran wie beispielsweise OmpF. Zusätzlich enthalten die Fraktionen die rekombinanten Proteine. Die Mikroröhren wurden IR-spektroskopisch untersucht **(****Fig. 6****).** Dazu werden die Membranen von rSllB/Lic93f_PIHis und rSllB-N/Lic704f_PI_GFP zum Zweck der kompletten Lipidextraktion mit Chloroform und Methanol behandelt (Bligh and Dyer 1959), wobei zunächst alle Proteine ausgefällt werden, welche anschließend als dritte Fraktion untersucht werden können. Nach der Phasentrennung werden beide Phasen, die untere Lipidphase und die obere lipidfreie Phase, durch IR-Spektroskopie analysiert **(****Fig. 6****).** Die Ergebnisse der IR-Spektroskopie stützen die Ergebnisse der SDS-PAGE, dass die Mikroröhren zum Großteil aus den rekombinanten Proteinen bestehen. Außerdem enthalten die Mikroröhren bis dato nicht identifizierte Lipide und andere nicht näher bestimmte Materialien.

### Ausführungsbeispiel 4: Mikroskopische Untersuchung der Mikroröhren

AFM-Proben werden auf Siliziumträger aufgebracht und getrocknet, und mit dem Cantilever AC240 (Olympus Optical, Europe; GmbH, Hamburg) im AC-Modus an der Luft analysiert.

Für die Untersuchung per Transmissionselektronenmikroskopie werden die Proben wie zuvor beschrieben in einem Volumen von 200 µl gut gewachsener Zellkultur in 1 x PBS und durch Zugabe von Osmiumtetroxid und Uranylacetat als Kontrastmittel behandelt (Spurr, 1969). Nach der Fixierung, Kontrastierung und Dehydrierung werden die Zellen in Epoxidharz (Serva Modified Spurr) entsprechend der Vorschriften des Herstellers eingebettet. Von den Proben werden mit dem Mikrotom Leica Ultracut UCR (Leica Microsystems GmbH, Wetzlar, Germany) und dem Diatome Diamont Messer (Diatome, Hatfield, Panama) Dünnschnitte mit einer Dicke von 50-300 nm hergestellt. Die luftgetrockneten Schnitte werden trocken gelagert.

*E. coli* BI21(DE3) Zellen, die kein SIIB-kodierendes Plasmid enthalten, sind durchschnittlich 1-2 µm lang und 0.5-0.7 µm breit **(****Fig. 2** **C, D).** Im Vergleich bilden, *E. coli* BI21(DE3), die SllB exprimieren, längere Einzelzellen aus, die im Durchschnitt 4-5 µm lang und 0.8-1 µm breit sind, und die Länge eines Zellkomplexes beträgt zwischen 5 und 200 µm **(****Fig. 1 A-C****).** Diese Zellklone bilden die erfindungsgemäßen Mikroröhren, die mit den Zellen wachsen und nicht durch die Zellteilung geteilt werden.

In der frühen exponentiellen Wachstumsphase formen die Zellen von nicht-transgenen *E. coli* BI21(DE3) ebenfalls solche speziellen Mikroröhren, die jedoch beim Erreichen der stationären Phase disaggregieren, wobei die einzelnen Zellen zurückbleiben. Im Vergleich dazu bilden die transgenen *E. coli* BI21(DE3) dieselben langen Mikroröhren um jede Zelle, die nicht durch Zellteilung geteilt werden. Die Mikroröhren der transgenen *E. coli* BI21(DE3) bleiben jedoch bei Erreichen der stationären Phase stabil.

### In der Beschreibung zitierte Nichtpatentliteratur

Battistuzzi G, Esan GJF, Fasuan FA, Modiano G, Luzzatto L (1977) Comparison of GdA und GdB Activities in Nigerians. A Study of the Variation of the G6PD Activity. Am J Hum Genet 29:31-36
Bligh ED, Dyer WJ (1959) A rapid method of total lipid extraction and purification. Canad. J. Biochem. Physiol. 37, 911-917.
Laemmli UK (1970) Cleavage of structural proteins during assembly of the head of bacteriophage T4. Nature 227:680-685
Pollmann, K. u. a. (2005). Novel surface layer protein genes in Bacillus sphaericus associated with unusual isertium elements. Microbiology 151 (Pt9) 2961-2973.
Pollmann, K. u. a (2007). Construction of S-layer protein exhibiting modified self-assembling properties and enhanced metal binding capacities. Appl. Microbiol. Biotechnology 75 (5) 1079-1085.
Preusser, H. J. 1959. Form und Größe des Kernäquivalentes von Escherichia coli in Abhängigkeit von den Kulturbedingungen. Archives of Microbiology 33:105-123.
Sanger F., Nicklen S., Coulson A.R., 1977. DNA Sequenzing with Chain-Terminating Inhibitors. Proceedings of the National Academy of Sciences of the United States of America, Vol 74, No.12, pp. 5463-5467
Selenska-Pobell S., Panak P., Miteva V., Boudakov I., Bernhard G., Nitsche H. (1999) Selective accumulation of heavy metals by three indigenous Bacillus strains, B. cereus, B. megaterium and B. sphaericus, from drain waters of uranium waste pile. FEMS Microbiol Ecol 29:59-67
Sleytr, U.B. et al., (2007). S-layers as a tool kit for nanobiotechnological applications. FEMS Microbiol. Lett. 267(2) 131-144
Spurr AR (1969) A low-viscosity epoxy resin embedding medium for electron microscopy. J Ultrastruct.Res. 26: 31-43

## Patentansprüche

1. Mikroröhren mit einem Durchmesser von 0,4 µm bis 2 µm, umfassend Bestandteile der äußeren Membran von *E. coli* Zellen und rekombinant exprimierte S-Layer-Proteine.

2. Mikroröhren gemäß Anspruch 1, erhältlich durch die Expression von einer für ein S-Layer-Protein kodierenden Nukleinsäuresequenz oder einer Teilsequenz davon in *E*. *coli*-Zellen, wobei sich die Mikroröhren um die Zellen herum bilden und eine oder mehrere Zellen umschließen, wobei die Zellen ggf. anschließend entfernt werden.

3. Mikroröhren gemäß Anspruch 1 oder 2 mit einem Durchmesser von 0,5 µm bis 1 µm, und/oder einer Länge 5 µm bis 200 µm, bevorzugt von 10 µm und 100 µm, besonders bevorzugt von 20 µm bis 80 µm.

4. Mikroröhren gemäß einem der Ansprüche 1 bis 3, **dadurch gekennzeichnet, dass** die S-Layer-Proteine S-Layer-Proteine der Gattung *Lysinibacillus sphaericus* sind.

5. Mikroröhren gemäß einem der Ansprüche 1 bis 4, **dadurch gekennzeichnet, dass** die *E*. *coli-Zellen* vom Stamm *E. coli* BI21 (DE3) und/oder andere filamentbildende *E*. *coli*-Zellen sind.

6. Mikroröhren gemäß einem der Ansprüche 1 bis 5, **dadurch gekennzeichnet, dass** die S-Layer-Proteine mit Peptiden oder Proteinen fusioniert sind.

7. Mikroröhren gemäß einem der Ansprüche 1 bis 6, **dadurch gekennzeichnet, dass** die Röhren metallisiert sind.

8. Mikroröhren gemäß Anspruch 7, **dadurch gekennzeichnet, dass** die Röhren durch Inkubation in einer Metallsalzlösung und anschließende Zugabe eines Reduktionsmittels metallisiert werden.

9. Verfahren zur Herstellung von Mikroröhren mit den Schritten:
a.) Expression von S-Layer-Proteinen in *E*. coli-Zellen, wobei sich die Mikroröhren um die Zellen herum bilden und eine oder mehrere Zellen umschließen und
b.) ggf. Entfernung der *E*. *coli*-Zellen, bevorzugt durch Behandlung mit der wässrigen Lösung eines Mono- und/oder Disaccharids oder einer wässrigen Ethanollösung.

10. Verwendung einer Nukleinsäuresequenz mit der SEQ-ID No. 1, 3, 5 oder 7, einer Teilsequenz davon oder einer zu mindestens einer dieser Sequenzen homologe Sequenz mit mindestens 70 % Sequenzidentität, bevorzugt 80 % Sequenzidentität, besonders bevorzugt 90 % Sequenzidentität, zur Herstellung von biologischen Mikroröhren gemäß einem der Ansprüche 1 bis 9, wobei die Teilsequenz mindestens für einen der Bereiche, die von 217 bis 600 Aminosäuren oder von 824 Aminosäuren bis zum C-terminalen Ende einer der Sequenzen gemäß den SEQ ID No. 2, 4, 6 oder 8 reichen, codiert.

11. Verwendung von Mikroröhren gemäß einem der Ansprüche 1 bis 8 zur Bindung von Metall aus einer wässrigen Lösung, zur Herstellung funktionalisierter Schichten, als Carrier für Enzyme oder als Carrier für pharmazeutische Wirkstoffe.

12. Verwendung von Mikroröhren gemäß einem der Ansprüche 6 bis 8 als Katalysator.

13. Verwendung einer *E. coli-*Zelle umfassend eine Nukleinsäuresequenz ausgewählt aus SEQ-ID No. 1,3,5 oder 7, eine Teilsequenz davon oder eine zu mindestens einer dieser Sequenzen homologe Sequenz mit mindestens 70 % Sequenzidentität, bevorzugt 80 % Sequenzidentität, besonders bevorzugt 90 % Sequenzidentität zur Herstellung von biologischen Mikroröhren gemäß einem der Ansprüche 1 bis 9, wobei die Teilsequenz mindestens für einen der Bereiche, die von 217 bis 600 Aminosäuren oder von 824 Aminosäuren bis zum C-terminalen Ende einer der Sequenzen gemäß den SEQ ID No. 2, 4, 6 oder 8 reichen, codiert.

14. Verwendung einer *E*. *coli*-Zelle gemäß Anspruch 13 **dadurch gekennzeichnet, dass** die *E*. *coli*-Zelle vom Stamm *E*. *coli* BI21 (DE3) und/oder eine andere filamentbildende *E*. *coli-*Zelle ist.

## Claims

1. Microtubes having a diameter of from 0.4 µm to 2 µm, comprising components of the outer membrane of *E. coli* cells and recombinantly expressed S-layer proteins.

2. Microtubes according to claim 1, obtainable by means of the expression of a nucleic acid sequence coding for an S-Layer protein, or a partial sequence thereof in *E. coli* cells, wherein the microtubes form around the cells and encompass one or more cells, wherein the cells are then optionally removed.

3. Microtubes according to either claim 1 or claim 2 having a diameter of from 0.5 µm to 1 µm, and/or a length of from 5 µm to 200 µm, preferably of 10 µm and 100 µm, more preferably of from 20 µm to 80 µm.

4. Microtubes according to any of claims 1 to 3, **characterised in that** the S-layer proteins are S-layer proteins of the genus *Lysinibacillus sphaericus.*

5. Microtubes according to any of claims 1 to 4, **characterised in that** the *E*. *coli* cells are from the strain *E. coli* BI21 (DE3) and/or are other filament-forming *E. coli* cells.

6. Microtubes according to any of claims 1 to 5, **characterised in that** the S-layer proteins are fused with peptides or proteins.

7. Microtubes according to any of claims 1 to 6, **characterised in that** the tubes are metallised.

8. Microtubes according to claim 7, **characterised in that** the tubes are metallised by means of incubation in a metal salt solution and subsequent addition of a reducing agent.

9. Method for producing microtubes, comprising the steps of:
a) expressing S-layer proteins in *E. coli* cells, wherein the microtubes form around the cells and encompass one or more cells, and
b) optionally removing the *E. coli* cells, preferably by treatment with the aqueous solution of a monosaccharide and/or a disaccharide or an aqueous ethanol solution.

10. Use of a nucleic acid sequence of SEQ ID No. 1, 3, 5 or 7, a partial sequence thereof or a sequence that is homologous to at least one of said sequences having at least 70 % sequence identity, preferably 80 % sequence identity, more preferably 90 % sequence identity, for producing organic microtubes according to any of claims 1 to 9, wherein the partial sequence codes at least for one of the ranges which span from 217 to 600 amino acids or from 824 amino acids to the C-terminal end of one of the sequences according to SEQ ID No. 2, 4, 6 or 8.

11. Use of microtubes according to any of claims 1 to 8 for binding metals from an aqueous solution, for producing functionalised layers, as a carrier for enzymes or as a carrier for pharmaceutical active ingredients.

12. Use of microtubes according to any of claims 6 to 8 as a catalyst.

13. Use of an *E*. *coli* cell comprising a nucleic acid sequence selected from SEQ ID No. 1, 3, 5 or 7, a partial sequence thereof or a sequence that is homologous to at least one of said sequences having at least 70 % sequence identity, preferably 80 % sequence identity, more preferably 90 % sequence identity, for producing organic microtubes according to any of claims 1 to 9, wherein the partial sequence codes at least for one of the ranges which span from 217 to 600 amino acids or from 824 amino acids to the C-terminal end of one of the sequences according to SEQ ID No. 2, 4, 6 or 8.

14. Use of an *E*. *coli* cell according to claim 13, **characterised in that** the *E*. *coli* cell is from the strain *E. coli* BI21 (DE3) and/or is another filament-forming *E. coli* cell.

## Revendications

1. Microtubes avec un diamètre de 0,4 µm à 2 µm, comprenant des éléments de la membrane externe de cellules d'*E*. *coli* et des protéines à couche S exprimées de manière recombinante.

2. Microtubes selon la revendication 1, disponibles via l'expression d'une séquence d'acide nucléique codant pour une protéine à couche S ou d'une séquence partielle de celle-ci dans des cellules d'*E*. *coli,* dans lesquels les microtubes se forment autour des cellules et entourent une ou plusieurs cellules, dans lesquels les cellules le cas échéant sont ensuite éliminées.

3. Microtubes selon la revendication 1 ou 2 avec un diamètre de 0,5 µm à 1 µm, et/ou une longueur de 5 µm à 200 µm, de préférence de 10 µm et 100 µm, plus préférablement de 20 µm à 80 µm.

4. Microtubes selon l'une des revendications 1 à 3, **caractérisés en ce que** les protéines à couche S sont des protéines à couche S du genre *Lysinibacillus sphaericus.*

5. Microtubes selon l'une des revendications 1 à 4, **caractérisés en ce que** les cellules d'*E*. *coli* proviennent de la souche d'*E*. *coli* BI21 (DE3) et/ou sont d'autres cellules d'*E*. *coli* filamenteuses.

6. Microtubes selon l'une des revendications 1 à 5, **caractérisés en ce que** les protéines à couche S sont fusionnées avec des peptides ou des protéines.

7. Microtubes selon l'une des revendications 1 à 6, **caractérisés en ce que** les tubes sont métallisés.

8. Microtubes selon la revendication 7, **caractérisés en ce que** les tubes sont métallisés par incubation dans une solution de sels métalliques et ajout ultérieur d'un agent de réduction.

9. Procédé de fabrication de microtubes avec les étapes suivantes :
a) expression de protéines à couche S dans des cellules d'*E*. *coli,* les microtubes se formant autour des cellules et entourant une ou plusieurs cellules et
b) le cas échéant élimination des cellules d'*E*. *coli,* de préférence par traitement avec la solution aqueuse d'un monosaccharide et/ou d'un disaccharide ou d'une solution d'éthanol aqueuse.

10. Utilisation d'une séquence d'acide nucléique avec les SEQ ID N° 1, 3, 5 ou 7, d'une séquence partielle de celle-ci ou d'une séquence homologue à au moins l'une de ces séquences avec une identité de séquence d'au moins 70 %, de préférence une identité de séquence de 80 %, plus préférablement une identité de séquence de 90 %, pour la fabrication de microtubes biologiques selon l'une des revendications 1 à 9, dans laquelle la séquence partielle code au moins pour l'un des domaines qui vont de 217 à 600 acides aminés ou de 824 acides aminés jusqu'à l'extrémité C-terminale de l'une des séquences selon les SEQ ID N° 2, 4, 6 ou 8.

11. Utilisation de microtubes selon l'une des revendications 1 à 8 pour la liaison de métal provenant d'une solution aqueuse, pour la fabrication de couches fonctionnalisées, en tant que véhicule pour des enzymes ou en tant que véhicule pour des substances actives pharmaceutiques.

12. Utilisation de microtubes selon l'une des revendications 6 à 8 en tant que catalyseur.

13. Utilisation d'une cellule d'*E*. *coli* comprenant une séquence d'acide nucléique sélectionnée parmi les SEQ ID N° 1, 3, 5 ou 7, une séquence partielle de celle-ci ou une séquence homologue à au moins l'une de ces séquences avec une identité de séquence d'au moins 70 %, de préférence une identité de séquence de 80 %, plus préférablement une identité de séquence de 90 % pour la fabrication de microtubes biologiques selon l'une des revendications 1 à 9, dans laquelle la séquence partielle code au moins pour l'un des domaines qui vont de 217 à 600 acides aminés ou de 824 acides aminés jusqu'à l'extrémité C-terminale de l'une des séquences selon les SEQ ID N° 2, 4, 6 ou 8.

14. Utilisation d'une cellule d'*E*. *coli* selon la revendication 13, **caractérisée en ce que** la cellule d'*E*. *coli* provient d'une souche d'*E*. *coli* BI21 (DE3) et/ou est une autre cellule d'*E*. *coli* filamenteuse.
